# EUROPEAN PATENT APPLICATION

(11) **EP 1 529 537 A1**
(43) Date of publication of application: **11.05.2005**
(21) Application number: 03078217.1
(22) Date of filing: 10.10.2003
(51) Int. Cl.: A61K 51/04, C07D 231/12, C07D 231/14, A61P 35/00

(54) **Bifunctional tridentate pyrazolyl containing ligands for RE, TC and MN tricarbonyl complexes**

(71) Applicant: Mallinckrodt International Corporation, St. Louis, Missouri 63134 (US)
(72) Inventor: Santos, Isabel Rego, 1800 Lisboa (PT); Paulo, Antonio Manuel, 1900 Lisboa (PT); Vitor, Rute, 2685 Santa Iria Da Azoia (PT); Correia, Joao Domingos Galamba, 1070-373 Lisboa (PT); Alves, Susana, 2800-310 Almada (PT)
(74) Representative: Van Someren, Petronella F. H. M.

(57) **Abstract**

The present invention relates to new cheating agents to link biomolecules and carbonyl moieties for labeling with technetuim and rhenium. In particular the invention relates to bifunctional tridentate pyrazolyl-polyamines, pyrazolyl-aminothioethers, pyrazolyl-polythioethers, pyrazolyl-aminophosphines and pyrazolyl-thioetherphosphines which stabilize the moieties [M(CO)₃]⁺ (M = Re, Tc, Mn) and bind to biomolecules which accumulate in diseased tissues. The invention relates to the chelators as such, to chelators coupled to a biomolecule and to either of these complexed with carbonyl. In addition the invention relates to a kit for providing radiolabeled biomolecules and to the use of such radiolabeled molecules in diagnosis and therapy.

## Description

This invention lies in the field of radiopharmaceuticals and provides new chelating agents to link biomolecules and carbonyl moieties for labeling with technetium and rhenium. In particular the invention relates to bifunctional tridentate pyrazolyl-polyamines, pyrazolyl-aminothioethers, pyrazolyl-polythioethers, pyrazolyl-aminophosphines and pyrazolyl-thioetherphosphines which stabilize the moieties [M(CO)₃]⁺ (M = Re, Tc, Mn) and bind to biomolecules which accumulate in diseased tissues. The invention relates to the chelators as such, to chelators coupled to a biomolecule and to either of these complexed with carbonyl. In addition the invention relates to a kit for providing radiolabeled biomolecules and to the use of such radiolabeled molecules in diagnosis and therapy.

The diagnosis and therapy of cancer still needs a significant input from the chemical, radiochemical and pharmaceutical point of view. Tumour seeking compounds stable *in vitro* and *in vivo*, with high specific activity and specificity are still an important issue in the radiopharmaceutical field. Since the publication of international patents on [Re(CO)₃]⁺ and [Tc(CO)₃]⁺ [1] a significant interest has appeared in this oxidation state, which opens new perspectives on pharmaceutical and Nuclear Medicine fields. The search for new chelating agents is essential as they are determinant for the uptake of biological vectors. Several chelating agents have been described in patents [1, 2] and publications [3, 4, 5].

It is the object of the present invention to enlarge the family of bifunctional chelating agents.

This is achieved by the invention by chelating agents of the general formula: 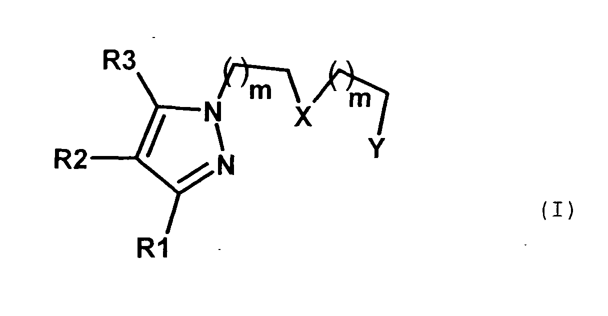 wherein
m is 0 or 1;
X is NR₄ or S;
Y is SR₅, NHR₅ or P(R₅)₂;
R₁ and R₃ are the same or different and are selected from H, alkyl or aryl;
R₂ is H, COOH, NHR₆ or (CH₂)ₙCOOR₆;
R₄ is H, alkyl, aryl, (CH₂)ₙCOOR₆ or (CH₂)ₙOR₆;
R₅ is H, alkyl, aryl, (CH₂)ₙCOOR₆ or (CH₂)ₙOR₆
R₆ is H, alkyl or aryl;
n is 1, 2, 3, 4, 5, 6, 7, 8, 9 or 10; and
when R₁= R₃=CH₃, R₂, R₄ and R₅ are not all three H.

These molecules combine two functions. One is for the stabilization of metal centers, including radioactive metals, and comprises different donor atom sets, and the other is a functional group for binding to the molecule of interest.

The alkyl is a C₁ alkyl, C₂ alkyl, C₃ alkyl, C₄ alkyl, C₅ alkyl or C₆ alkyl, in particular selected from methyl, ethyl, *n*-propyl, isopropyl, *n*-butyl, isobutyl, *s*-butyl, *t*-butyl, *n*-pentyl, isopentyl, neopentyl, *n*-hexyl, isohexyl (2-methylpentyl), neohexyl (2,2-dimethylbutyl), 3-methylpentyl, 2,3- dimethylbutyl.

The aryls are monocyclic, C₅-C₈, or polycyclic C₁₀-C₁₈, and are optionally substituted with alkyl, carboxy, oxo, amino, alkoxy or aldehyde groups.
n is 2, 3, 4, 5 or 6 and preferably 2, 3 or 4.

The chelating agent is for example a pyrazolyl-polyamine of the general formula: 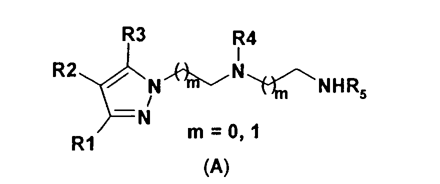 wherein R₁, R₂, R₃, R₄ and R₅ are as defined above.

Alternatively, the chelating agent is a pyrazolyl-aminothioether of the general formula: 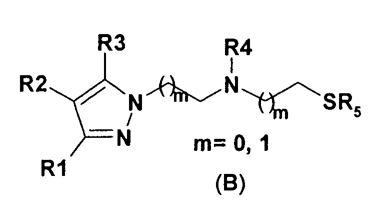 wherein R₁, R₂, R₃, R₄ and R₅ are as defined above.

In yet another embodiment the chelating agent is a pyrazolyl-polythioether of the general formula: 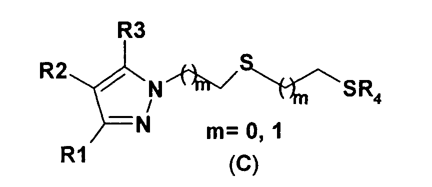 wherein R₁, R₂, R₃, R₄ and R₅ are as defined above.

In yet another embodiment the chelating agent is a pyrazolyl-aminophosphine of the general formula : 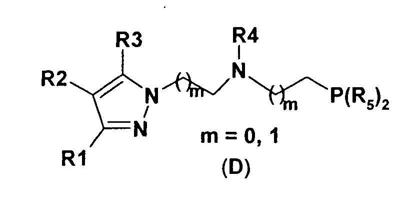 wherein R₁, R₂, R₃, R₄ and R₅ are as defined above.

In a further embodiment the chelating agent is a pyrazolyl-thioetherphosphine of the general formula : 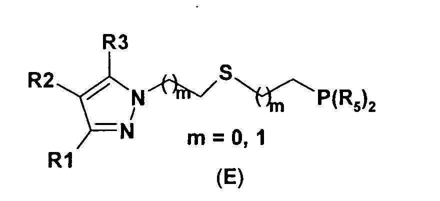 wherein R₁, R₂, R₃, R₄ and R₅ are as defined above.

The invention provides more particularly chelating agents of formula I, wherein X and Y are N, R₆ is H, C₁ alkyl, C₂ alkyl, C₃ alkyl, C₄ alkyl, C₅ alkyl or C₆ alkyl, monocyclic aryls, preferably phenyl or benzyl, or polycyclic C₁₀-C₁₈ aryls, optionally substituted with alkyl, carboxy, oxo amino, alkoxy or aldehyde groups, or a biomolecule and R₁, R₃, R₃, R₄ and R₅ are as listed in Table 1.

In another embodiment the invention relates to chelating agents of formula I, wherein X and Y are S, R₆ is H, C₁ alkyl, C₂ alkyl, C₃ alkyl, C₄ alkyl, C₅ alkyl or C₆ alkyl, monocyclic aryls, preferably phenyl or benzyl, or polycyclic C₁₀-C₁₈ aryls, optionally substituted with alkyl, carboxy, oxo amino, alkoxy or aldehyde groups, or a biomolecule and R₁, R₂, R₃, R₄ and R₅ are as listed in Table 1.

In yet another embodiment chelating agents of formula I are provided, wherein X is N and Y is S, R₆ is H, C₁ alkyl, C₂ alkyl, C₃ alkyl, C₄ alkyl, C₅ alkyl or C₆ alkyl, monocyclic aryls, preferably phenyl or benzyl, or polycyclic C₁₀-C₁₈ aryls, optionally substituted with alkyl, carboxy, oxo amino, alkoxy or aldehyde groups, or a biomolecule and R₁, R₂, R₃, R₄ and R₅ are as listed in Table 1.

According to a further aspect thereof the invention relates to chelating agents of formula I, wherein X is S and Y are N, R₆ is H, C₁ alkyl, C₂ alkyl, C₃ alkyl, C₄ alkyl, C₅ alkyl or C₆ alkyl, monocyclic aryls, preferably phenyl or benzyl, or polycyclic C₁₀-C₁₈ aryls, optionally substituted with alkyl, carboxy, oxo amino, alkoxy or aldehyde groups, or a biomolecule and R₁, R₂, R₃, R₄ and R₅ are as listed in Table 1.

According to another aspect of the invention, chelating agents of formula I are provided, wherein X is N and Y is P, R₆ is H, C₁ alkyl, C₂ alkyl, C₃ alkyl, C₄ alkyl, C₅ alkyl or C₆ alkyl, monocyclic aryls, preferably phenyl or benzyl, or polycyclic C₁₀-C₁₈ aryls, optionally substituted with alkyl, carboxy, oxo amino, alkoxy or aldehyde groups, or a biomolecule and R₁, R₂, R₃, R₄ and R₅ are as listed in Table 1.

In another embodiment the invention relates to chelating agents of formula I, wherein X is S and Y is P, R₆ is H, C₁ alkyl, C₂ alkyl, C₃ alkyl, C₄ alkyl, C₅ alkyl or C₆ alkyl, monocyclic aryls, preferably phenyl or benzyl, or polycyclic C₁₀-C₁₈ aryls, optionally substituted with alkyl, carboxy, oxo amino, alkoxy or aldehyde groups, or a biomolecule and R₁, R₂, R₃, R₄ and R₅ are as listed in Table 1.

The chelating agents of the invention are particularly suited to link biomolecules with carbonyl moieties in order to arrive at labeled biomolecules having a high specificity for the target. In formula I R₆ can thus be a biomolecule.

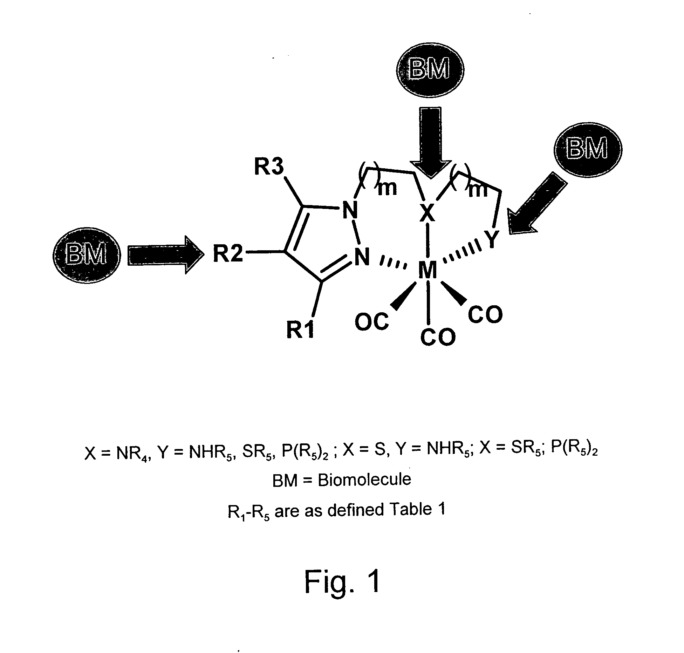

The possible positions of the biomolecules (BM) are shown in **Fig. 1.**

The biomolecule can be anything that is useful in the treatment and diagnosis of tumors and can be coupled to the chelators of the invention. The skilled person will be able to establish for which biomolecules the chelators of the invention can be used. In particular the biomolecule is selected from amino acids, peptides, proteins, oligonucleotides, polynucleotides, sugars.

More specifically, the biomolecule is selected from the group consisting of antibodies, ligands of tumor receptors, such as CCK, thioglucose, glucosamine, somatostatin, neurotensin, bombesin, CCK, annexin, interleukins, growth factors, steroid hormones and molecules binding to GPIIb/IIIa receptors. Other biomolecules can be glucose, thioglucose, neurotransmitters, inhibitors of the tyrosine kinase activity such as benzothiopyranones, anilinophthalimides, quinazolines, pyridopyrimidines and pyrrolopyrimidines.

Particular agents of the invention are the following:

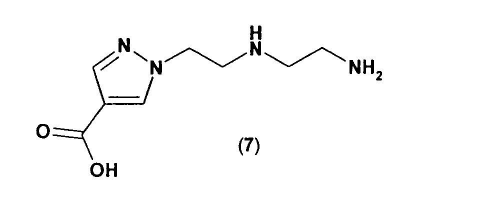

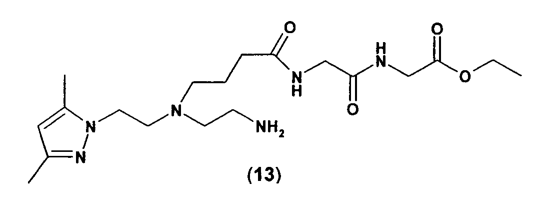

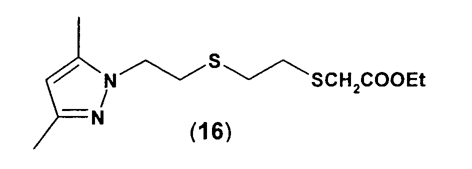

All of the chelating agents, either with or without a biomolecule coupled thereto can be complexed with a carbonyl moiety of the formula [M(CO)₃]⁺, wherein M is rhenium (Re), technetium (Tc) or Manganese (Mn).

The chelating agents of the invention are molecules according to formula I wherein X and Y can be either N and N, N and S, S and N, S and S, N and P, or S and P. Each of these combinations can be combined with various combinations of R₁, R₂, R₃, R₄ and R₅. All possible combinations of R₁, R₂, R₃, R₄ and R₅ are listed in **Table 1.** In **Table 1** alkyl is a C₁ alkyl, C₂ alkyl, C₃ alkyl, C₄ alkyl, C₅ alkyl or C₆ alkyl, in particular selected from methyl, ethyl, *n*-propyl, isopropyl, *n*-butyl, isobutyl, *s*-butyl, t-butyl, *n*-pentyl, isopentyl, neopentyl, *n*-hexyl, isohexyl (2-methylpentyl), neohexyl (2,2-dimethylbutyl), 3-methylpentyl, 2,3-dimethylbutyl; the aryl is monocyclic, C₅-C₈, or polycyclic, C₁₀-C₁₈, and optionally substituted with alkyl, carboxy, oxo, amino, alkoxy or aldehyde groups and is in particular phenyl or benzyl, and n is 1, 2, 3, 4, 5, 6, 7, 8, 9 or 10. R₆ is H, alkyl, aryl or a biomolecule as defined above. Substituting each of the above variables into the table will give all compounds of claim 1 that are herewith disclosed.

**Table 1**

| **R1** | **R2** | **R3** | **R4** | **R5** |
|---|---|---|---|---|
| H | H | H | H | H |
| H | H | H | H | alkyl |
| H | H | H | H | aryl |
| H | H | H | H | (CH₂)ₙCOOR₆ |
| H | H | H | H | (CH₂)ₙOR₆ |
| H | H | H | alkyl | H |
| H | H | H | alkyl | alkyl |
| H | H | H | alkyl | aryl |
| H | H | H | alkyl | (CH₂)ₙCOOR₆ |
| H | H | H | alkyl | (CH₂)ₙOR₆ |
| H | H | H | aryl | H |
| H | H | H | aryl | alkyl |
| H | H | H | aryl | aryl |
| H | H | H | aryl | (CH₂)ₙCOOR₆ |
| H | H | H | aryl | (CH₂)ₙOR₆ |
| H | H | H | (CH₂)ₙCOOR₆ | H |
| H | H | H | (CH₂)ₙCOOR₆ | alkyl |
| H | H | H | (CH₂)ₙCOOR₆ | aryl |
| H | H | H | (CH₂)ₙCOOR₆ | (CH₂)ₙCOOR₆ |
| H | H | H | (CH₂)ₙCOOR₆ | (CH₂)ₙOR₆ |
| H | H | H | (CH₂)ₙOR₆ | H |
| H | H | H | (CH₂)ₙOR₆ | alkyl |
| H | H | H | (CH₂)ₙOR₆ | aryl |
| H | H | H | (CH₂)ₙOR₆ | (CH₂)ₙCOOR₆ |
| H | H | H | (CH₂)ₙOR₆ | (CH₂)ₙOR₆ |
| H | H | alkyl | H | H |
| H | H | alkyl | H | alkyl |
| H | H | alkyl | H | aryl |
| H | H | alkyl | H | (CH₂)ₙCOOR₆ |
| H | H | alkyl | H | (CH₂)ₙOR₆ |
| H | H | alkyl | alkyl | H |
| H | H | alkyl | alkyl | alkyl |
| H | H | alkyl | alkyl | aryl |
| H | H | alkyl | alkyl | (CH₂)ₙCOOR₆ |
| H | H | alkyl | alkyl | (CH₂)ₙOR₆ |
| H | H | alkyl | aryl | H |
| H | H | alkyl | aryl | alkyl |
| H | H | alkyl | aryl | aryl |
| H | H | alkyl | aryl | (CH₂)ₙCOOR₆ |
| H | H | alkyl | aryl | (CH₂)ₙOR₆ |
| H | H | alkyl | (CH₂)ₙCOOR₆ | H |
| H | H | alkyl | (CH₂)ₙCOOR₆ | alkyl |
| H | H | alkyl | (CH₂)ₙCOOR₆ | aryl |
| H | H | alkyl | (CH₂)ₙCOOR₆ | (CH₂)ₙCOOR₆ |
| H | H | alkyl | (CH₂)ₙCOOR₆ | (CH₂)ₙOR₆ |
| H | H | alkyl | (CH₂)ₙOR₆ | H |
| H | H | alkyl | (CH₂)ₙOR₆ | alkyl |
| H | H | alkyl | (CH₂)ₙOR₆ | aryl |
| H | H | alkyl | (CH₂)ₙOR₆ | (CH₂)ₙCOOR₆ |
| H | H | alkyl | (CH₂)ₙOR₆ | (CH₂)ₙOR₆ |
| H | H | aryl | H | H |
| H | H | aryl | H | alkyl |
| H | H | aryl | H | aryl |
| H | H | aryl | H | (CH₂)ₙCOOR₆ |
| H | H | aryl | H | (CH₂)ₙOR₆ |
| H | H | aryl | alkyl | H |
| H | H | aryl | alkyl | alkyl |
| H | H | aryl | alkyl | aryl |
| H | H | aryl | alkyl | (CH₂)ₙCOOR₆ |
| H | H | aryl | alkyl | (CH₂)ₙOR₆ |
| H | H | aryl | aryl | H |
| H | H | aryl | aryl | alkyl |
| H | H | aryl | aryl | aryl |
| H | H | aryl | aryl | (CH₂)ₙCOOR₆ |
| H | H | aryl | aryl | (CH₂)ₙOR₆ |
| H | H | aryl | (CH₂)ₙCOOR₆ | H |
| H | H | aryl | (CH₂)ₙCOOR₆ | alkyl |
| H | H | aryl | (CH₂)ₙCOOR₆ | aryl |
| H | H | aryl | (CH₂)ₙCOOR₆ | (CH₂)ₙCOOR₆ |
| H | H | aryl | (CH₂)ₙCOOR₆ | (CH₂)ₙOR₆ |
| H | H | aryl | (CH₂)ₙOR₆ | H |
| H | H | aryl | (CH₂)ₙOR₆ | alkyl |
| H | H | aryl | (CH₂)ₙOR₆ | aryl |
| H | H | aryl | (CH₂)ₙOR₆ | (CH₂)ₙCOOR₆ |
| H | H | aryl | (CH₂)ₙOR₆ | (CH₂)ₙOR₆ |
| H | COOH | H | H | H |
| H | COOH | H | H | alkyl |
| H | COOH | H | H | aryl |
| H | COOH | H | H | (CH₂)ₙCOOR₆ |
| H | COOH | H | H | (CH₂)ₙOR₆ |
| H | COOH | H | alkyl | H |
| H | COOH | H | alkyl | alkyl |
| H | COOH | H | alkyl | aryl |
| H | COOH | H | alkyl | (CH₂)ₙCOOR₆ |
| H | COOH | H | alkyl | (CH₂)ₙOR₆ |
| H | COOH | H | aryl | H |
| H | COOH | H | aryl | alkyl |
| H | COOH | H | aryl | aryl |
| H | COOH | H | aryl | (CH₂)ₙCOOR₆ |
| H | COOH | H | aryl | (CH₂)ₙOR₆ |
| H | COOH | H | (CH₂)ₙCOOR₆ | H |
| H | COOH | H | (CH₂)ₙCOOR₆ | alkyl |
| H | COOH | H | (CH₂)ₙCOOR₆ | aryl |
| H | COOH | H | (CH₂)ₙCOOR₆ | (CH₂)ₙCOOR₆ |
| H | COOH | H | (CH₂)ₙCOOR₆ | (CH₂)ₙOR₆ |
| H | COOH | H | (CH₂)ₙOR₆ | H |
| H | COOH | H | (CH₂)ₙOR₆ | alkyl |
| H | COOH | H | (CH₂)ₙOR₆ | aryl |
| H | COOH | H | (CH₂)ₙOR₆ | (CH₂)ₙCOOR₆ |
| H | COOH | H | (CH₂)ₙOR₆ | (CH₂)ₙOR₆ |
| H | COOH | alkyl | H | H |
| H | COOH | alkyl | H | alkyl |
| H | COOH | alkyl | H | aryl |
| H | COOH | alkyl | H | (CH₂)ₙCOOR₆ |
| H | COOH | alkyl | H | (CH₂)ₙOR₆ |
| H | COOH | alkyl | alkyl | H |
| H | COOH | alkyl | alkyl | alkyl |
| H | COOH | alkyl | alkyl | aryl |
| H | COOH | alkyl | alkyl | (CH₂)ₙCOOR₆ |
| H | COOH | alkyl | alkyl | (CH₂)ₙOR₆ |
| H | COOH | alkyl | aryl | H |
| H | COOH | alkyl | aryl | alkyl |
| H | COOH | alkyl | aryl | aryl |
| H | COOH | alkyl | aryl | (CH₂)ₙCOOR₆ |
| H | COOH | alkyl | aryl | (CH₂)ₙOR₆ |
| H | COOH | alkyl | (CH₂)ₙCOOR₆ | H |
| H | COOH | alkyl | (CH₂)ₙCOOR₆ | alkyl |
| H | COOH | alkyl | (CH₂)ₙCOOR₆ | aryl |
| H | COOH | alkyl | (CH₂)ₙCOOR₆ | (CH₂)ₙCOOR₆ |
| H | COOH | alkyl | (CH₂)ₙCOOR₆ | (CH₂)ₙOR₆ |
| H | COOH | alkyl | (CH₂)ₙOR₆ | H |
| H | COOH | alkyl | (CH₂)ₙOR₆ | alkyl |
| H | COOH | alkyl | (CH₂)ₙOR₆ | aryl |
| H | COOH | alkyl | (CH₂)ₙOR₆ | (CH₂)ₙCOOR₆ |
| H | COOH | alkyl | (CH₂)ₙOR₆ | (CH₂)ₙOR₆ |
| H | COOH | aryl | H | H |
| H | COOH | aryl | H | alkyl |
| H | COOH | aryl | H | aryl |
| H | COOH | aryl | H | (CH₂)ₙCOOR₆ |
| H | COOH | aryl | H | (CH₂)ₙOR₆ |
| H | COOH | aryl | alkyl | H |
| H | COOH | aryl | alkyl | alkyl |
| H | COOH | aryl | alkyl | aryl |
| H | COOH | aryl | alkyl | (CH₂)ₙCOOR₆ |
| H | COOH | aryl | alkyl | (CH₂)ₙOR₆ |
| H | COOH | aryl | aryl | H |
| H | COOH | aryl | aryl | alkyl |
| H | COOH | aryl | aryl | aryl |
| H | COOH | aryl | aryl | (CH₂)ₙCOOR₆ |
| H | COOH | aryl | aryl | (CH₂)ₙOR₆ |
| H | COOH | aryl | (CH₂)ₙCOOR₆ | H |
| H | COOH | aryl | (CH₂)ₙCOOR₆ | alkyl |
| H | COOH | aryl | (CH₂)ₙCOOR₆ | aryl |
| H | COOH | aryl | (CH₂)ₙCOOR₆ | (CH₂)ₙCOOR₆ |
| H | COOH | aryl | (CH₂)ₙCOOR₆ | (CH₂)ₙOR₆ |
| H | COOH | aryl | (CH₂)ₙOR₆ | H |
| H | COOH | aryl | (CH₂)ₙOR₆ | alkyl |
| H | COOH | aryl | (CH₂)ₙOR₆ | aryl |
| H | COOH | aryl | (CH₂)ₙOR₆ | (CH₂)ₙCOOR₆ |
| H | COOH | aryl | (CH₂)ₙOR₆ | (CH₂)ₙOR₆ |
| H | NHR₆ | H | H | H |
| H | NHR₆ | H | H | alkyl |
| H | NHR₆ | H | H | aryl |
| H | NHR₆ | H | H | (CH₂)ₙCOOR₆ |
| H | NHR₆ | H | H | (CH₂)ₙOR₆ |
| H | NHR₆ | H | alkyl | H |
| H | NHR₆ | H | alkyl | alkyl |
| H | NHR₆ | H | alkyl | aryl |
| H | NHR₆ | H | alkyl | (CH₂)ₙCOOR₆ |
| H | NHR₆ | H | alkyl | (CH₂)ₙOR₆ |
| H | NHR₆ | H | aryl | H |
| H | NHR₆ | H | aryl | alkyl |
| H | NHR₆ | H | aryl | aryl |
| H | NHR₆ | H | aryl | (CH₂)ₙCOOR₆ |
| H | NHR₆ | H | aryl | (CH₂)ₙOR₆ |
| H | NHR₆ | H | (CH₂)ₙCOOR₆ | H |
| H | NHR₆ | H | (CH₂)ₙCOOR₆ | alkyl |
| H | NHR₆ | H | (CH₂)ₙCOOR₆ | aryl |
| H | NHR₆ | H | (CH₂)ₙCOOR₆ | (CH₂)ₙCOOR₆ |
| H | NHR₆ | H | (CH₂)ₙCOOR₆ | (CH₂)ₙOR₆ |
| H | NHR₆ | H | (CH₂)ₙOR₆ | H |
| H | NHR₆ | H | (CH₂)ₙOR₆ | alkyl |
| H | NHR₆ | H | (CH₂)ₙOR₆ | aryl |
| H | NHR₆ | H | (CH₂)ₙOR₆ | (CH₂)ₙCOOR₆ |
| H | NHR₆ | H | (CH₂)ₙOR₆ | (CH₂)ₙOR₆ |
| H | NHR₆ | alkyl | H | H |
| H | NHR₆ | alkyl | H | alkyl |
| H | NHR₆ | alkyl | H | aryl |
| H | NHR₆ | alkyl | H | (CH₂)ₙCOOR₆ |
| H | NHR₆ | alkyl | H | (CH₂)ₙOR₆ |
| H | NHR₆ | alkyl | alkyl | H |
| H | NHR₆ | alkyl | alkyl | alkyl |
| H | NHR₆ | alkyl | alkyl | aryl |
| H | NHR₆ | alkyl | alkyl | (CH₂)ₙCOOR₆ |
| H | NHR₆ | alkyl | alkyl | (CH₂)ₙOR₆ |
| H | NHR₆ | alkyl | aryl | H |
| H | NHR₆ | alkyl | aryl | alkyl |
| H | NHR₆ | alkyl | aryl | aryl |
| H | NHR₆ | alkyl | aryl | (CH₂)ₙCOOR₆ |
| H | NHR₆ | alkyl | aryl | (CH₂)ₙOR₆ |
| H | NHR₆ | alkyl | (CH₂)ₙCOOR₆ | H |
| H | NHR₆ | alkyl | (CH₂)ₙCOOR₆ | alkyl |
| H | NHR₆ | alkyl | (CH₂)ₙCOOR₆ | aryl |
| H | NHR₆ | alkyl | (CH₂)ₙCOOR₆ | (CH₂)ₙCOOR₆ |
| H | NHR₆ | alkyl | (CH₂)ₙCOOR₆ | (CH₂)ₙOR₆ |
| H | NHR₆ | alkyl | (CH₂)ₙOR₆ | H |
| H | NHR₆ | alkyl | (CH₂)ₙOR₆ | alkyl |
| H | NHR₆ | alkyl | (CH₂)ₙOR₆ | aryl |
| H | NHR₆ | alkyl | (CH₂)ₙOR₆ | (CH₂)ₙCOOR₆ |
| H | NHR₆ | alkyl | (CH₂)ₙOR₆ | (CH₂)ₙOR₆ |
| H | NHR₆ | aryl | H | H |
| H | NHR₆ | aryl | H | alkyl |
| H | NHR₆ | aryl | H | aryl |
| H | NHR₆ | aryl | H | (CH₂)ₙCOOR₆ |
| H | NHR₆ | aryl | H | (CH₂)ₙOR₆ |
| H | NHR₆ | aryl | alkyl | H |
| H | NHR₆ | aryl | alkyl | alkyl |
| H | NHR₆ | aryl | alkyl | aryl |
| H | NHR₆ | aryl | alkyl | (CH₂)ₙCOOR₆ |
| H | NHR₆ | aryl | alkyl | (CH₂)ₙOR₆ |
| H | NHR₆ | aryl | aryl | H |
| H | NHR₆ | aryl | aryl | alkyl |
| H | NHR₆ | aryl | aryl | aryl |
| H | NHR₆ | aryl | aryl | (CH₂)ₙCOOR₆ |
| H | NHR₆ | aryl | aryl | (CH₂)ₙOR₆ |
| H | NHR₆ | aryl | (CH₂)ₙCOOR₆ | H |
| H | NHR₆ | aryl | (CH₂)ₙCOOR₆ | alkyl |
| H | NHR₆ | aryl | (CH₂)ₙCOOR₆ | aryl |
| H | NHR₆ | aryl | (CH₂)ₙCOOR₆ | (CH₂)ₙCOOR₆ |
| H | NHR₆ | aryl | (CH₂)ₙCOOR₆ | (CH₂)ₙOR₆ |
| H | NHR₆ | aryl | (CH₂)ₙOR₆ | H |
| H | NHR₆ | aryl | (CH₂)ₙOR₆ | alkyl |
| H | NHR₆ | aryl | (CH₂)ₙOR₆ | aryl |
| H | NHR₆ | aryl | (CH₂)ₙOR₆ | (CH₂)ₙCOOR₆ |
| H | NHR₆ | aryl | (CH₂)ₙOR₆ | (CH₂)ₙOR₆ |
| H | (CH₂)ₙCOOR₆ | H | H | H |
| H | (CH₂)ₙCOOR₆ | H | H | alkyl |
| H | (CH₂)ₙCOOR₆ | H | H | aryl |
| H | (CH₂)ₙCOOR₆ | H | H | (CH₂)ₙCOOR₆ |
| H | (CH₂)ₙCOOR₆ | H | H | (CH₂)ₙOR₆ |
| H | (CH₂)ₙCOOR₆ | H | alkyl | H |
| H | (CH₂)ₙCOOR₆ | H | alkyl | alkyl |
| H | (CH₂)ₙCOOR₆ | H | alkyl | aryl |
| H | (CH₂)ₙCOOR₆ | H | alkyl | (CH₂)ₙCOOR₆ |
| H | (CH₂)ₙCOOR₆ | H | alkyl | (CH₂)ₙOR₆ |
| H | (CH₂)ₙCOOR₆ | H | aryl | H |
| H | (CH₂)ₙCOOR₆ | H | aryl | alkyl |
| H | (CH₂)ₙCOOR₆ | H | aryl | aryl |
| H | (CH₂)ₙCOOR₆ | H | aryl | (CH₂)ₙCOOR₆ |
| H | (CH₂)ₙCOOR₆ | H | aryl | (CH₂)ₙOR₆ |
| H | (CH₂)ₙCOOR₆ | H | (CH₂)ₙCOOR₆ | H |
| H | (CH₂)ₙCOOR₆ | H | (CH₂)ₙCOOR₆ | alkyl |
| H | (CH₂)ₙCOOR₆ | H | (CH₂)ₙCOOR₆ | aryl |
| H | (CH₂)ₙCOOR₆ | H | (CH₂)ₙCOOR₆ | (CH₂)ₙCOOR₆ |
| H | (CH₂)ₙCOOR₆ | H | (CH₂)ₙCOOR₆ | (CH₂)ₙOR₆ |
| H | (CH₂)ₙCOOR₆ | H | (CH₂)ₙOR₆ | H |
| H | (CH₂)ₙCOOR₆ | H | (CH₂)ₙOR₆ | alkyl |
| H | (CH₂)ₙCOOR₆ | H | (CH₂)ₙOR₆ | aryl |
| H | (CH₂)ₙCOOR₆ | H | (CH₂)ₙOR₆ | (CH₂)ₙCOOR₆ |
| H | (CH₂)ₙCOOR₆ | H | (CH₂)ₙOR₆ | (CH₂)ₙOR₆ |
| H | (CH₂)ₙCOOR₆ | alkyl | H | H |
| H | (CH₂)ₙCOOR₆ | alkyl | H | alkyl |
| H | (CH₂)ₙCOOR₆ | alkyl | H | aryl |
| H | (CH₂)ₙCOOR₆ | alkyl | H | (CH₂)ₙCOOR₆ |
| H | (CH₂)ₙCOOR₆ | alkyl | H | (CH₂)ₙOR₆ |
| H | (CH₂)ₙCOOR₆ | alkyl | alkyl | H |
| H | (CH₂)ₙCOOR₆ | alkyl | alkyl | alkyl |
| H | (CH₂)ₙCOOR₆ | alkyl | alkyl | aryl |
| H | (CH₂)ₙCOOR₆ | alkyl | alkyl | (CH₂)ₙCOOR₆ |
| H | (CH₂)ₙCOOR₆ | alkyl | alkyl | (CH₂)ₙOR₆ |
| H | (CH₂)ₙCOOR₆ | alkyl | aryl | H |
| H | (CH₂)ₙCOOR₆ | alkyl | aryl | alkyl |
| H | (CH₂)ₙCOOR₆ | al kyl | aryl | aryl |
| H | (CH₂)ₙCOOR₆ | alkyl | aryl | (CH₂)ₙCOOR₆ |
| H | (CH₂)ₙCOOR₆ | alkyl | aryl | (CH₂)ₙOR₆ |
| H | (CH₂)ₙCOOR₆ | alkyl | (CH₂)ₙCOOR₆ | H |
| H | (CH₂)ₙCOOR₆ | alkyl | (CH₂)ₙCOOR₆ | alkyl |
| H | (CH₂)ₙCOOR₆ | alkyl | (CH₂)ₙCOOR₆ | aryl |
| H | (CH₂)ₙCOOR₆ | alkyl | (CH₂)ₙCOOR₆ | (CH₂)ₙCOOR₆ |
| H | (CH₂)ₙCOOR₆ | alkyl | (CH₂)ₙCOOR₆ | (CH₂)ₙOR₆ |
| H | (CH₂)ₙCOOR₆ | alkyl | (CH₂)ₙOR₆ | H |
| H | (CH₂)ₙCOOR₆ | alkyl | (CH₂)ₙOR₆ | alkyl |
| H | (CH₂)ₙCOOR₆ | alkyl | (CH₂)ₙOR₆ | aryl |
| H | (CH₂)ₙCOOR₆ | alkyl | (CH₂)ₙOR₆ | (CH₂)ₙCOOR₆ |
| H | (CH₂)ₙCOOR₆ | alkyl | (CH₂)ₙOR₆ | (CH₂)ₙOR₆ |
| H | (CH₂)ₙCOOR₆ | aryl | H | H |
| H | (CH₂)ₙCOOR₆ | aryl | H | alkyl |
| H | (CH₂)ₙCOOR₆ | aryl | H | aryl |
| H | (CH₂)ₙCOOR₆ | aryl | H | (CH₂)ₙCOOR₆ |
| H | (CH₂)ₙCOOR₆ | aryl | H | (CH₂)ₙOR₆ |
| H | (CH₂)ₙCOOR₆ | aryl | alkyl | H |
| H | (CH₂)ₙCOOR₆ | aryl | alkyl | alkyl |
| H | (CH₂)ₙCOOR₆ | aryl | alkyl | aryl |
| H | (CH₂)ₙCOOR₆ | aryl | alkyl | (CH₂)ₙCOOR₆ |
| H | (CH₂)ₙCOOR₆ | aryl | alkyl | (CH₂)ₙOR₆ |
| H | (CH₂)ₙCOOR₆ | aryl | aryl | H |
| H | (CH₂)ₙCOOR₆ | aryl | aryl | alkyl |
| H | (CH₂)ₙCOOR₆ | aryl | aryl | aryl |
| H | (CH₂)ₙCOOR₆ | aryl | aryl | (CH₂)ₙCOOR₆ |
| H | (CH₂)ₙCOOR₆ | aryl | aryl | (CH₂)ₙOR₆ |
| H | (CH₂)ₙCOOR₆ | aryl | (CH₂)ₙCOOR₆ | H |
| H | (CH₂)ₙCOOR₆ | aryl | (CH₂)ₙCOOR₆ | alkyl |
| H | (CH₂)ₙCOOR₆ | aryl | (CH₂)ₙCOOR₆ | aryl |
| H | (CH₂)ₙCOOR₆ | aryl | (CH₂)ₙCOOR₆ | (CH₂)ₙCOOR₆ |
| H | (CH₂)ₙCOOR₆ | aryl | (CH₂)ₙCOOR₆ | (CH₂)ₙOR₆ |
| H | (CH₂)ₙCOOR₆ | aryl | (CH₂)ₙOR₆ | H |
| H | (CH₂)ₙCOOR₆ | aryl | (CH₂)ₙOR₆ | alkyl |
| H | (CH₂)ₙCOOR₆ | aryl | (CH₂)ₙOR₆ | aryl |
| H | (CH₂)ₙCOOR₆ | aryl | (CH₂)ₙOR₆ | (CH₂)ₙCOOR₆ |
| H | (CH₂)ₙCOOR₆ | aryl | (CH₂)ₙOR₆ | (CH₂)ₙOR₆ |
| alkyl | H | H | H | H |
| alkyl | H | H | H | alkyl |
| alkyl | H | H | H | aryl |
| alkyl | H | H | H | (CH₂)ₙCOOR₆ |
| alkyl | H | H | H | (CH₂)ₙOR₆ |
| alkyl | H | H | alkyl | H |
| alkyl | H | H | alkyl | alkyl |
| alkyl | H | H | alkyl | aryl |
| alkyl | H | H | alkyl | (CH₂)ₙCOOR₆ |
| alkyl | H | H | alkyl | (CH₂)ₙOR₆ |
| alkyl | H | H | aryl | H |
| alkyl | H | H | aryl | alkyl |
| alkyl | H | H | aryl | aryl |
| alkyl | H | H | aryl | (CH₂)ₙCOOR₆ |
| alkyl | H | H | aryl | (CH₂)ₙOR₆ |
| alkyl | H | H | (CH₂)ₙCOOR₆ | H |
| alkyl | H | H | (CH₂)ₙCOOR₆ | alkyl |
| alkyl | H | H | (CH₂)ₙCOOR₆ | aryl |
| alkyl | H | H | (CH₂)ₙCOOR₆ | (CH₂)ₙCOOR₆ |
| alkyl | H | H | (CH₂)ₙCOOR₆ | (CH₂)ₙOR₆ |
| alkyl | H | H | (CH₂)ₙOR₆ | H |
| alkyl | H | H | (CH₂)ₙOR₆ | alkyl |
| alkyl | H | H | (CH₂)ₙOR₆ | aryl |
| alkyl | H | H | (CH₂)ₙOR₆ | (CH₂)ₙCOOR₆ |
| alkyl | H | H | (CH₂)ₙOR₆ | (CH₂)ₙOR₆ |
| alkyl | H | alkyl | H | H |
| alkyl | H | alkyl | H | alkyl |
| alkyl | H | alkyl | H | aryl |
| alkyl | H | alkyl | H | (CH₂)ₙCOOR₆ |
| alkyl | H | alkyl | H | (CH₂)ₙOR₆ |
| alkyl | H | alkyl | alkyl | H |
| alkyl | H | alkyl | alkyl | alkyl |
| alkyl | H | alkyl | alkyl | aryl |
| alkyl | H | alkyl | alkyl | (CH₂)ₙCOOR₆ |
| alkyl | H | alkyl | alkyl | (CH₂)ₙOR₆ |
| alkyl | H | alkyl | aryl | H |
| alkyl | H | alkyl | aryl | alkyl |
| alkyl | H | alkyl | aryl | aryl |
| alkyl | H | alkyl | aryl | (CH₂)ₙCOOR₆ |
| alkyl | H | alkyl | aryl | (CH₂)ₙOR₆ |
| alkyl | H | alkyl | (CH₂)ₙCOOR₆ | H |
| alkyl | H | alkyl | (CH₂)ₙCOOR₆ | alkyl |
| alkyl | H | alkyl | (CH₂)ₙCOOR₆ | aryl |
| alkyl | H | alkyl | (CH₂)ₙCOOR₆ | (CH₂)ₙCOOR₆ |
| alkyl | H | alkyl | (CH₂)ₙCOOR₆ | (CH₂)ₙOR₆ |
| alkyl | H | alkyl | (CH₂)ₙOR₆ | H |
| alkyl | H | alkyl | (CH₂)ₙOR₆ | alkyl |
| alkyl | H | alkyl | (CH₂)ₙOR₆ | aryl |
| alkyl | H | alkyl | (CH₂)ₙOR₆ | (CH₂)ₙCOOR₆ |
| alkyl | H | alkyl | (CH₂)ₙOR₆ | (CH₂)ₙOR₆ |
| alkyl | H | aryl | H | H |
| alkyl | H | aryl | H | alkyl |
| alkyl | H | aryl | H | aryl |
| alkyl | H | aryl | H | (CH₂)ₙCOOR₆ |
| alkyl | H | aryl | H | (CH₂)ₙOR₆ |
| alkyl | H | aryl | alkyl | H |
| alkyl | H | aryl | alkyl | alkyl |
| alkyl | H | aryl | alkyl | aryl |
| alkyl | H | aryl | alkyl | (CH₂)ₙCOOR₆ |
| alkyl | H | aryl | alkyl | (CH₂)ₙOR₆ |
| alkyl | H | aryl | aryl | H |
| alkyl | H | aryl | aryl | alkyl |
| alkyl | H | aryl | aryl | aryl |
| alkyl | H | aryl | aryl | (CH₂)ₙCOOR₆ |
| alkyl | H | aryl | aryl | (CH₂)ₙOR₆ |
| alkyl | H | aryl | (CH₂)ₙCOOR₆ | H |
| alkyl | H | aryl | (CH₂)ₙCOOR₆ | alkyl |
| alkyl | H | aryl | (CH₂)ₙCOOR₆ | aryl |
| alkyl | H | aryl | (CH₂)ₙCOOR₆ | (CH₂)ₙCOOR₆ |
| alkyl | H | aryl | (CH₂)ₙCOOR₆ | (CH₂)ₙOR₆ |
| alkyl | H | aryl | (CH₂)ₙOR₆ | H |
| alkyl | H | aryl | (CH₂)ₙOR₆ | alkyl |
| alkyl | H | aryl | (CH₂)ₙOR₆ | aryl |
| alkyl | H | aryl | (CH₂)ₙOR₆ | (CH₂)ₙCOOR₆ |
| alkyl | H | aryl | (CH₂)ₙOR₆ | (CH₂)ₙOR₆ |
| alkyl | COOH | H | H | H |
| alkyl | COOH | H | H | alkyl |
| alkyl | COOH | H | H | aryl |
| alkyl | COOH | H | H | (CH₂)ₙCOOR₆ |
| alkyl | COOH | H | H | (CH₂)ₙOR₆ |
| alkyl | COOH | H | alkyl | H |
| alkyl | COOH | H | alkyl | alkyl |
| alkyl | COOH | H | alkyl | aryl |
| alkyl | COOH | H | alkyl | (CH₂)ₙCOOR₆ |
| alkyl | COOH | H | alkyl | (CH₂)ₙOR₆ |
| alkyl | COOH | H | aryl | H |
| alkyl | COOH | H | aryl | alkyl |
| alkyl | COOH | H | aryl | aryl |
| alkyl | COOH | H | aryl | (CH₂)ₙCOOR₆ |
| alkyl | COOH | H | aryl | (CH₂)ₙOR₆ |
| alkyl | COOH | H | (CH₂)ₙCOOR₆ | H |
| alkyl | COOH | H | (CH₂)ₙCOOR₆ | alkyl |
| alkyl | COOH | H | (CH₂)ₙCOOR₆ | aryl |
| alkyl | COOH | H | (CH₂)ₙCOOR₆ | (CH₂)ₙCOOR₆ |
| alkyl | COOH | H | (CH₂)ₙCOOR₆ | (CH₂)ₙOR₆ |
| alkyl | COOH | H | (CH₂)ₙOR₆ | H |
| alkyl | COOH | H | (CH₂)ₙOR₆ | alkyl |
| alkyl | COOH | H | (CH₂)ₙOR₆ | aryl |
| alkyl | COOH | H | (CH₂)ₙOR₆ | (CH₂)ₙCOOR₆ |
| alkyl | COOH | H | (CH₂)ₙOR₆ | (CH₂)ₙOR₆ |
| alkyl | COOH | alkyl | H | H |
| alkyl | COOH | alkyl | H | alkyl |
| alkyl | COOH | alkyl | H | aryl |
| alkyl | COOH | alkyl | H | (CH₂)ₙCOOR₆ |
| alkyl | COOH | alkyl | H | (CH₂)ₙOR₆ |
| alkyl | COOH | alkyl | alkyl | H |
| alkyl | COOH | alkyl | alkyl | alkyl |
| alkyl | COOH | alkyl | alkyl | aryl |
| alkyl | COOH | alkyl | alkyl | (CH₂)ₙCOOR₆ |
| alkyl | COOH | alkyl | alkyl | (CH₂)ₙOR₆ |
| alkyl | COOH | alkyl | aryl | H |
| alkyl | COOH | alkyl | aryl | alkyl |
| alkyl | COOH | alkyl | aryl | aryl |
| alkyl | COOH | alkyl | aryl | (CH₂)ₙCOOR₆ |
| alkyl | COOH | alkyl | aryl | (CH₂)ₙOR₆ |
| alkyl | COOH | alkyl | (CH₂)ₙCOOR₆ | H |
| alkyl | COOH | alkyl | (CH₂)ₙCOOR₆ | alkyl |
| alkyl | COOH | alkyl | (CH₂)ₙCOOR₆ | aryl |
| alkyl | COOH | alkyl | (CH₂)ₙCOOR₆ | (CH₂)ₙCOOR₆ |
| alkyl | COOH | alkyl | (CH₂)ₙCOOR₆ | (CH₂)ₙOR₆ |
| alkyl | COOH | alkyl | (CH₂)ₙOR₆ | H |
| alkyl | COOH | alkyl | (CH₂)ₙOR₆ | alkyl |
| alkyl | COOH | alkyl | (CH₂)ₙOR₆ | aryl |
| alkyl | COOH | alkyl | (CH₂)ₙOR₆ | (CH₂)ₙCOOR₆ |
| alkyl | COOH | alkyl | (CH₂)ₙOR₆ | (CH₂)ₙOR₆ |
| alkyl | COOH | aryl | H | H |
| alkyl | COOH | aryl | H | alkyl |
| alkyl | COOH | aryl | H | aryl |
| alkyl | COOH | aryl | H | (CH₂)ₙCOOR₆ |
| alkyl | COOH | aryl | H | (CH₂)ₙOR₆ |
| alkyl | COOH | aryl | alkyl | H |
| alkyl | COOH | aryl | alkyl | alkyl |
| alkyl | COOH | aryl | alkyl | aryl |
| alkyl | COOH | aryl | alkyl | (CH₂)ₙCOOR₆ |
| alkyl | COOH | aryl | alkyl | (CH₂)ₙOR₆ |
| alkyl | COOH | aryl | aryl | H |
| alkyl | COOH | aryl | aryl | alkyl |
| alkyl | COOH | aryl | aryl | aryl |
| alkyl | COOH | aryl | aryl | (CH₂)ₙCOOR₆ |
| alkyl | COOH | aryl | aryl | (CH₂)ₙOR₆ |
| alkyl | COOH | aryl | (CH₂)ₙCOOR₆ | H |
| alkyl | COOH | aryl | (CH₂)ₙCOOR₆ | alkyl |
| alkyl | COOH | aryl | (CH₂)ₙCOOR₆ | aryl |
| alkyl | COOH | aryl | (CH₂)ₙCOOR₆ | (CH₂)ₙCOOR₆ |
| alkyl | COOH | aryl | (CH₂)ₙCOOR₆ | (CH₂)ₙOR₆ |
| alkyl | COOH | aryl | (CH₂)ₙOR₆ | H |
| alkyl | COOH | aryl | (CH₂)ₙOR₆ | alkyl |
| alkyl | COOH | aryl | (CH₂)ₙOR₆ | aryl |
| alkyl | COOH | aryl | (CH₂)ₙOR₆ | (CH₂)ₙCOOR₆ |
| alkyl | COOH | aryl | (CH₂)ₙOR₆ | (CH₂)ₙOR₆ |
| alkyl | NHR₆ | H | H | H |
| alkyl | NHR₆ | H | H | alkyl |
| alkyl | NHR₆ | H | H | aryl |
| alkyl | NHR₆ | H | H | (CH₂)ₙCOOR₆ |
| alkyl | NHR₆ | H | H | (CH₂)ₙOR₆ |
| alkyl | NHR₆ | H | alkyl | H |
| alkyl | NHR₆ | H | alkyl | alkyl |
| alkyl | NHR₆ | H | alkyl | aryl |
| alkyl | NHR₆ | H | alkyl | (CH₂)ₙCOOR₆ |
| alkyl | NHR₆ | H | alkyl | (CH₂)ₙOR₆ |
| alkyl | NHR₆ | H | aryl | H |
| alkyl | NHR₆ | H | aryl | alkyl |
| alkyl | NHR₆ | H | aryl | aryl |
| alkyl | NHR₆ | H | aryl | (CH₂)ₙCOOR₆ |
| alkyl | NHR₆ | H | aryl | (CH₂)ₙOR₆ |
| alkyl | NHR₆ | H | (CH₂)ₙCOOR₆ | H |
| alkyl | NHR₆ | H | (CH₂)ₙCOOR₆ | alkyl |
| alkyl | NHR₆ | H | (CH₂)ₙCOOR₆ | aryl |
| alkyl | NHR₆ | H | (CH₂)ₙCOOR₆ | (CH₂)ₙCOOR₆ |
| alkyl | NHR₆ | H | (CH₂)ₙCOOR₆ | (CH₂)ₙOR₆ |
| alkyl | NHR₆ | H | (CH₂)ₙOR₆ | H |
| alkyl | NHR₆ | H | (CH₂)ₙOR₆ | alkyl |
| alkyl | NHR₆ | H | (CH₂)ₙOR₆ | aryl |
| alkyl | NHR₆ | H | (CH₂)ₙOR₆ | (CH₂)ₙCOOR₆ |
| alkyl | NHR₆ | H | (CH₂)ₙOR₆ | (CH₂)ₙOR₆ |
| alkyl | NHR₆ | alkyl | H | H |
| alkyl | NHR₆ | alkyl | H | alkyl |
| alkyl | NHR₆ | alkyl | H | aryl |
| alkyl | NHR₆ | alkyl | H | (CH₂)ₙCOOR₆ |
| alkyl | NHR₆ | alkyl | H | (CH₂)ₙOR₆ |
| alkyl | NHR₆ | alkyl | alkyl | H |
| alkyl | NHR₆ | alkyl | alkyl | alkyl |
| alkyl | NHR₆ | alkyl | alkyl | aryl |
| alkyl | NHR₆ | alkyl | alkyl | (CH₂)ₙCOOR₆ |
| alkyl | NHR₆ | alkyl | alkyl | (CH₂)ₙOR₆ |
| alkyl | NHR₆ | alkyl | aryl | H |
| alkyl | NHR₆ | alkyl | aryl | alkyl |
| alkyl | NHR₆ | alkyl | aryl | aryl |
| alkyl | NHR₆ | alkyl | aryl | (CH₂)ₙCOOR₆ |
| alkyl | NHR₆ | alkyl | aryl | (CH₂)ₙOR₆ |
| alkyl | NHR₆ | alkyl | (CH₂)ₙCOOR₆ | H |
| alkyl | NHR₆ | alkyl | (CH₂)ₙCOOR₆ | alkyl |
| alkyl | NHR₆ | alkyl | (CH₂)ₙCOOR₆ | aryl |
| alkyl | NHR₆ | alkyl | (CH₂)ₙCOOR₆ | (CH₂)ₙCOOR₆ |
| alkyl | NHR₆ | alkyl | (CH₂)ₙCOOR₆ | (CH₂)ₙOR₆ |
| alkyl | NHR₆ | alkyl | (CH₂)ₙOR₆ | H |
| alkyl | NHR₆ | alkyl | (CH₂)ₙOR₆ | alkyl |
| alkyl | NHR₆ | alkyl | (CH₂)ₙOR₆ | aryl |
| alkyl | NHR₆ | alkyl | (CH₂)ₙOR₆ | (CH₂)ₙCOOR₆ |
| alkyl | NHR₆ | alkyl | (CH₂)ₙOR₆ | (CH₂)ₙOR₆ |
| alkyl | NHR₆ | aryl | H | H |
| alkyl | NHR₆ | aryl | H | alkyl |
| alkyl | NHR₆ | aryl | H | aryl |
| alkyl | NHR₆ | aryl | H | (CH₂)ₙCOOR₆ |
| alkyl | NHR₆ | aryl | H | (CH₂)ₙOR₆ |
| alkyl | NHR₆ | aryl | alkyl | H |
| alkyl | NHR₆ | aryl | alkyl | alkyl |
| alkyl | NHR₆ | aryl | alkyl | aryl |
| alkyl | NHR₆ | aryl | alkyl | (CH₂)ₙCOOR₆ |
| alkyl | NHR₆ | aryl | alkyl | (CH₂)ₙOR₆ |
| alkyl | NHR₆ | aryl | aryl | H |
| alkyl | NHR₆ | aryl | aryl | alkyl |
| alkyl | NHR₆ | aryl | aryl | aryl |
| alkyl | NHR₆ | aryl | aryl | (CH₂)ₙCOOR₆ |
| alkyl | NHR₆ | aryl | aryl | (CH₂)ₙOR₆ |
| alkyl | NHR₆ | aryl | (CH₂)ₙCOOR₆ | H |
| alkyl | NHR₆ | aryl | (CH₂)ₙCOOR₆ | alkyl |
| alkyl | NHR₆ | aryl | (CH₂)ₙCOOR₆ | aryl |
| alkyl | NHR₆ | aryl | (CH₂)ₙCOOR₆ | (CH₂)ₙCOOR₆ |
| alkyl | NHR₆ | aryl | (CH₂)ₙCOOR₆ | (CH₂)ₙOR₆ |
| alkyl | NHR₆ | aryl | (CH₂)ₙOR₆ | H |
| alkyl | NHR₆ | aryl | (CH₂)ₙOR₆ | alkyl |
| alkyl | NHR₆ | aryl | (CH₂)ₙOR₆ | aryl |
| alkyl | NHR₆ | aryl | (CH₂)ₙOR₆ | (CH₂)ₙCOOR₆ |
| alkyl | NHR₆ | aryl | (CH₂)ₙOR₆ | (CH₂)ₙOR₆ |
| alkyl | (CH₂)ₙCOOR₆ | H | H | H |
| alkyl | (CH₂)ₙCOOR₆ | H | H | alkyl |
| alkyl | (CH₂)ₙCOOR₆ | H | H | aryl |
| alkyl | (CH₂)ₙCOOR₆ | H | H | (CH₂)ₙCOOR₆ |
| alkyl | (CH₂)ₙCOOR₆ | H | H | (CH₂)ₙOR₆ |
| alkyl | (CH₂)ₙCOOR₆ | H | alkyl | H |
| alkyl | (CH₂)ₙCOOR₆ | H | alkyl | alkyl |
| alkyl | (CH₂)ₙCOOR₆ | H | alkyl | aryl |
| alkyl | (CH₂)ₙCOOR₆ | H | alkyl | (CH₂)ₙCOOR₆ |
| alkyl | (CH₂)ₙCOOR₆ | H | alkyl | (CH₂)ₙOR₆ |
| alkyl | (CH₂)ₙCOOR₆ | H | aryl | H |
| alkyl | (CH₂)ₙCOOR₆ | H | aryl | alkyl |
| alkyl | (CH₂)ₙCOOR₆ | H | aryl | aryl |
| alkyl | (CH₂)ₙCOOR₆ | H | aryl | (CH₂)ₙCOOR₆ |
| alkyl | (CH₂)ₙCOOR₆ | H | aryl | (CH₂)ₙOR₆ |
| alkyl | (CH₂)ₙCOOR₆ | H | (CH₂)ₙCOOR₆ | H |
| alkyl | (CH₂)ₙCOOR₆ | H | (CH₂)ₙCOOR₆ | alkyl |
| alkyl | (CH₂)ₙCOOR₆ | H | (CH₂)ₙCOOR₆ | aryl |
| alkyl | (CH₂)ₙCOOR₆ | H | (CH₂)ₙCOOR₆ | (CH₂)ₙCOOR₆ |
| alkyl | (CH₂)ₙCOOR₆ | H | (CH₂)ₙCOOR₆ | (CH₂)ₙOR₆ |
| alkyl | (CH₂)ₙCOOR₆ | H | (CH₂)ₙOR₆ | H |
| alkyl | (CH₂)ₙCOOR₆ | H | (CH₂)ₙOR₆ | alkyl |
| alkyl | (CH₂)ₙCOOR₆ | H | (CH₂)ₙOR₆ | aryl |
| alkyl | (CH₂)ₙCOOR₆ | H | (CH₂)ₙOR₆ | (CH₂)ₙCOOR₆ |
| alkyl | (CH₂)ₙCOOR₆ | H | (CH₂)ₙOR₆ | (CH₂)ₙOR₆ |
| alkyl | (CH₂)ₙCOOR₆ | alkyl | H | H |
| alkyl | (CH₂)ₙCOOR₆ | alkyl | H | alkyl |
| alkyl | (CH₂)ₙCOOR₆ | alkyl | H | aryl |
| alkyl | (CH₂)ₙCOOR₆ | alkyl | H | (CH₂)ₙCOOR₆ |
| alkyl | (CH₂)ₙCOOR₆ | alkyl | H | (CH₂)ₙOR₆ |
| alkyl | (CH₂)ₙCOOR₆ | alkyl | alkyl | H |
| alkyl | (CH₂)ₙCOOR₆ | alkyl | alkyl | alkyl |
| al kyl | (CH₂)ₙCOOR₆ | al kyl | alkyl | aryl |
| alkyl | (CH₂)ₙCOOR₆ | alkyl | alkyl | (CH₂)ₙCOOR₆ |
| alkyl | (CH₂)ₙCOOR₆ | alkyl | alkyl | (CH₂)ₙOR₆ |
| alkyl | (CH₂)ₙCOOR₆ | alkyl | aryl | H |
| alkyl | (CH₂)ₙCOOR₆ | alkyl | aryl | alkyl |
| alkyl | (CH₂)ₙCOOR₆ | alkyl | aryl | aryl |
| alkyl | (CH₂)ₙCOOR₆ | alkyl | aryl | (CH₂)ₙCOOR₆ |
| alkyl | (CH₂)ₙCOOR₆ | alkyl | aryl | (CH₂)ₙOR₆ |
| alkyl | (CH₂)ₙCOOR₆ | alkyl | (CH₂)ₙCOOR₆ | H |
| alkyl | (CH₂)ₙCOOR₆ | alkyl | (CH₂)ₙCOOR₆ | alkyl |
| alkyl | (CH₂)ₙCOOR₆ | alkyl | (CH₂)ₙCOOR₆ | aryl |
| alkyl | (CH₂)ₙCOOR₆ | alkyl | (CH₂)ₙCOOR₆ | (CH₂)ₙCOOR₆ |
| alkyl | (CH₂)ₙCOOR₆ | alkyl | (CH₂)ₙCOOR₆ | (CH₂)ₙOR₆ |
| alkyl | (CH₂)ₙCOOR₆ | alkyl | (CH₂)ₙOR₆ | H |
| alkyl | (CH₂)ₙCOOR₆ | alkyl | (CH₂)ₙOR₆ | alkyl |
| alkyl | (CH₂)ₙCOOR₆ | alkyl | (CH₂)ₙOR₆ | aryl |
| alkyl | (CH₂)ₙCOOR₆ | alkyl | (CH₂)ₙOR₆ | (CH₂)ₙCOOR₆ |
| alkyl | (CH₂)ₙCOOR₆ | alkyl | (CH₂)ₙCOR₆ | (CH₂)ₙOR₆ |
| alkyl | (CH₂)ₙCOOR₆ | aryl | H | H |
| alkyl | (CH₂)ₙCOOR₆ | aryl | H | alkyl |
| alkyl | (CH₂)ₙCOOR₆ | aryl | H | aryl |
| alkyl | (CH₂)ₙCOOR₆ | aryl | H | (CH₂)ₙCOOR₆ |
| alkyl | (CH₂)ₙCOOR₆ | aryl | H | (CH₂)ₙOR₆ |
| alkyl | (CH₂)ₙCOOR₆ | aryl | alkyl | H |
| alkyl | (CH₂)ₙCOOR₆ | aryl | alkyl | alkyl |
| alkyl | (CH₂)ₙCOOR₆ | aryl | alkyl | aryl |
| alkyl | (CH₂)ₙCOOR₆ | aryl | alkyl | (CH₂)ₙCOOR₆ |
| alkyl | (CH₂)ₙCOOR₆ | aryl | alkyl | (CH₂)ₙOR₆ |
| alkyl | (CH₂)ₙCOOR₆ | aryl | aryl | H |
| alkyl | (CH₂)ₙCOOR₆ | aryl | aryl | alkyl |
| alkyl | (CH₂)ₙCOOR₆ | aryl | aryl | aryl |
| alkyl | (CH₂)ₙCOOR₆ | aryl | aryl | (CH₂)ₙCOOR₆ |
| alkyl | (CH₂)ₙCOOR₆ | aryl | aryl | (CH₂)ₙOR₆ |
| alkyl | (CH₂)ₙCOOR₆ | aryl | (CH₂)ₙCOOR₆ | H |
| alkyl | (CH₂)ₙCOOR₆ | aryl | (CH₂)ₙCOOR₆ | alkyl |
| alkyl | (CH₂)ₙCOOR₆ | aryl | (CH₂)ₙCOOR₆ | aryl |
| alkyl | (CH₂)ₙCOOR₆ | aryl | (CH₂)ₙCOOR₆ | (CH₂)ₙCOOR₆ |
| alkyl | (CH₂)ₙCOOR₆ | aryl | (CH₂)ₙCOOR₆ | (CH₂)ₙOR₆ |
| alkyl | (CH₂)ₙCOOR₆ | aryl | (CH₂)ₙOR₆ | H |
| alkyl | (CH₂)ₙCOOR₆ | aryl | (CH₂)ₙOR₆ | alkyl |
| alkyl | (CH₂)ₙCOOR₆ | aryl | (CH₂)ₙOR₆ | aryl |
| alkyl | (CH₂)ₙCOOR₆ | aryl | (CH₂)ₙOR₆ | (CH₂)ₙCOOR₆ |
| alkyl | (CH₂)ₙCOOR₆ | aryl | (CH₂)ₙOR₆ | (CH₂)ₙOR₆ |
| aryl | H | H | H | H |
| aryl | H | H | H | alkyl |
| aryl | H | H | H | aryl |
| aryl | H | H | H | (CH₂)ₙCOOR₆ |
| aryl | H | H | H | (CH₂)ₙOR₆ |
| aryl | H | H | alkyl | H |
| aryl | H | H | alkyl | alkyl |
| aryl | H | H | alkyl | aryl |
| aryl | H | H | alkyl | (CH₂)ₙCOOR₆ |
| aryl | H | H | alkyl | (CH₂)ₙOR₆ |
| aryl | H | H | aryl | H |
| aryl | H | H | aryl | alkyl |
| aryl | H | H | aryl | aryl |
| aryl | H | H | aryl | (CH₂)ₙCOOR₆ |
| aryl | H | H | aryl | (CH₂)ₙOR₆ |
| aryl | H | H | (CH₂)ₙCOOR₆ | H |
| aryl | H | H | (CH₂)ₙCOOR₆ | alkyl |
| aryl | H | H | (CH₂)ₙCOOR₆ | aryl |
| aryl | H | H | (CH₂)ₙCOOR₆ | (CH₂)ₙCOOR₆ |
| aryl | H | H | (CH₂)ₙCOOR₆ | (CH₂)ₙOR₆ |
| aryl | H | H | (CH₂)ₙOR₆ | H |
| aryl | H | H | (CH₂)ₙOR₆ | alkyl |
| aryl | H | H | (CH₂)ₙOR₆ | aryl |
| aryl | H | H | (CH₂)ₙOR₆ | (CH₂)ₙCOOR₆ |
| aryl | H | H | (CH₂)ₙOR₆ | (CH₂)ₙOR₆ |
| aryl | H | alkyl | H | H |
| aryl | H | alkyl | H | alkyl |
| aryl | H | alkyl | H | aryl |
| aryl | H | alkyl | H | (CH₂)ₙCOOR₆ |
| aryl | H | alkyl | H | (CH₂)ₙOR₆ |
| aryl | H | alkyl | alkyl | H |
| aryl | H | alkyl | alkyl | alkyl |
| aryl | H | alkyl | alkyl | aryl |
| aryl | H | alkyl | alkyl | (CH₂)ₙCOOR₆ |
| aryl | H | alkyl | alkyl | (CH₂)ₙOR₆ |
| aryl | H | alkyl | aryl | H |
| aryl | H | alkyl | aryl | alkyl |
| aryl | H | alkyl | aryl | aryl |
| aryl | H | alkyl | aryl | (CH₂)ₙCOOR₆ |
| aryl | H | alkyl | aryl | (CH₂)ₙOR₆ |
| aryl | H | alkyl | (CH₂)ₙCOOR₆ | H |
| aryl | H | alkyl | (CH₂)ₙCOOR₆ | alkyl |
| aryl | H | alkyl | (CH₂)ₙCOOR₆ | aryl |
| aryl | H | alkyl | (CH₂)ₙCOOR₆ | (CH₂)ₙCOOR₆ |
| aryl | H | alkyl | (CH₂)ₙCOOR₆ | (CH₂)ₙOR₆ |
| aryl | H | alkyl | (CH₂)ₙOR₆ | H |
| aryl | H | alkyl | (CH₂)ₙOR₆ | alkyl |
| aryl | H | alkyl | (CH₂)ₙOR₆ | aryl |
| aryl | H | alkyl | (CH₂)ₙOR₆ | (CH₂)ₙCOOR₆ |
| aryl | H | alkyl | (CH₂)ₙOR₆ | (CH₂)ₙOR₆ |
| aryl | H | aryl | H | H |
| aryl | H | aryl | H | alkyl |
| aryl | H | aryl | H | aryl |
| aryl | H | aryl | H | (CH₂)ₙCOOR₆ |
| aryl | H | aryl | H | (CH₂)ₙOR₆ |
| aryl | H | aryl | alkyl | H |
| aryl | H | aryl | alkyl | alkyl |
| aryl | H | aryl | alkyl | aryl |
| aryl | H | aryl | alkyl | (CH₂)ₙCOOR₆ |
| aryl | H | aryl | alkyl | (CH₂)ₙOR₆ |
| aryl | H | aryl | aryl | H |
| aryl | H | aryl | aryl | alkyl |
| aryl | H | aryl | aryl | aryl |
| aryl | H | aryl | aryl | (CH₂)ₙCOOR₆ |
| aryl | H | aryl | aryl | (CH₂)ₙOR₆ |
| aryl | H | aryl | (CH₂)ₙCOOR₆ | H |
| aryl | H | aryl | (CH₂)ₙCOOR₆ | alkyl |
| aryl | H | aryl | (CH₂)ₙCOOR₆ | aryl |
| aryl | H | aryl | (CH₂)ₙCOOR₆ | (CH₂)ₙCOOR₆ |
| aryl | H | aryl | (CH₂)ₙCOOR₆ | (CH₂)ₙOR₆ |
| aryl | H | aryl | (CH₂)ₙOR₆ | H |
| aryl | H | aryl | (CH₂)ₙOR₆ | alkyl |
| aryl | H | aryl | (CH₂)ₙOR₆ | aryl |
| aryl | H | aryl | (CH₂)ₙOR₆ | (CH₂)ₙCOOR₆ |
| aryl | H | aryl | (CH₂)ₙOR₆ | (CH₂)ₙOR₆ |
| aryl | COOH | H | H | H |
| aryl | COOH | H | H | alkyl |
| aryl | COOH | H | H | aryl |
| aryl | COOH | H | H | (CH₂)ₙCOOR₆ |
| aryl | COOH | H | H | (CH₂)ₙOR₆ |
| aryl | COOH | H | alkyl | H |
| aryl | COOH | H | alkyl | alkyl |
| aryl | COOH | H | alkyl | aryl |
| aryl | COOH | H | alkyl | (CH₂)ₙCOOR₆ |
| aryl | COOH | H | alkyl | (CH₂)ₙOR₆ |
| aryl | COOH | H | aryl | H |
| aryl | COOH | H | aryl | alkyl |
| aryl | COOH | H | aryl | aryl |
| aryl | COOH | H | aryl | (CH₂)ₙCOOR₆ |
| aryl | COOH | H | aryl | (CH₂)ₙOR₆ |
| aryl | COOH | H | (CH₂)ₙCOOR₆ | H |
| aryl | COOH | H | (CH₂)ₙCOOR₆ | alkyl |
| aryl | COOH | H | (CH₂)ₙCOOR₆ | aryl |
| aryl | COOH | H | (CH₂)ₙCOOR₆ | (CH₂)ₙCOOR₆ |
| aryl | COOH | H | (CH₂)ₙCOOR₆ | (CH₂)ₙOR₆ |
| aryl | COOH | H | (CH₂)ₙOR₆ | H |
| aryl | COOH | H | (CH₂)ₙOR₆ | alkyl |
| aryl | COOH | H | (CH₂)ₙOR₆ | aryl |
| aryl | COOH | H | (CH₂)ₙOR₆ | (CH₂)ₙCOOR₆ |
| aryl | COOH | H | (CH₂)ₙOR₆ | (CH₂)ₙOR₆ |
| aryl | COOH | alkyl | H | H |
| aryl | COOH | alkyl | H | alkyl |
| aryl | COOH | alkyl | H | aryl |
| aryl | COOH | alkyl | H | (CH₂)ₙCOOR₆ |
| aryl | COOH | alkyl | H | (CH₂)ₙOR₆ |
| aryl | COOH | alkyl | alkyl | H |
| aryl | COOH | alkyl | alkyl | alkyl |
| aryl | COOH | alkyl | alkyl | aryl |
| aryl | COOH | alkyl | alkyl | (CH₂)ₙCOOR₆ |
| aryl | COOH | alkyl | alkyl | (CH₂)ₙOR₆ |
| aryl | COOH | alkyl | aryl | H |
| aryl | COOH | alkyl | aryl | alkyl |
| aryl | COOH | alkyl | aryl | aryl |
| aryl | COOH | alkyl | aryl | (CH₂)ₙCOOR₆ |
| aryl | COOH | alkyl | aryl | (CH₂)ₙOR₆ |
| aryl | COOH | alkyl | (CH₂)ₙCOOR₆ | H |
| aryl | COOH | alkyl | (CH₂)ₙCOOR₆ | alkyl |
| aryl | COOH | alkyl | (CH₂)ₙCOOR₆ | aryl |
| aryl | COOH | alkyl | (CH₂)ₙCOOR₆ | (CH₂)ₙCOOR₆ |
| aryl | COOH | alkyl | (CH₂)ₙCOOR₆ | (CH₂)ₙOR₆ |
| aryl | COOH | alkyl | (CH₂)ₙOR₆ | H |
| aryl | COOH | alkyl | (CH₂)ₙOR₆ | alkyl |
| aryl | COOH | alkyl | (CH₂)ₙOR₆ | aryl |
| aryl | COOH | alkyl | (CH₂)ₙOR₆ | (CH₂)ₙCOOR₆ |
| aryl | COOH | alkyl | (CH₂)ₙOR₆ | (CH₂)ₙOR₆ |
| aryl | COOH | aryl | H | H |
| aryl | COOH | aryl | H | alkyl |
| aryl | COOH | aryl | H | aryl |
| aryl | COOH | aryl | H | (CH₂)ₙCOOR₆ |
| aryl | COOH | aryl | H | (CH₂)ₙOR₆ |
| aryl | COOH | aryl | alkyl | H |
| aryl | COOH | aryl | alkyl | alkyl |
| aryl | COOH | aryl | alkyl | aryl |
| aryl | COOH | aryl | alkyl | (CH₂)ₙCOOR₆ |
| aryl | COOH | aryl | alkyl | (CH₂)ₙOR₆ |
| aryl | COOH | aryl | aryl | H |
| aryl | COOH | aryl | aryl | alkyl |
| aryl | COOH | aryl | aryl | aryl |
| aryl | COOH | aryl | aryl | (CH₂)ₙCOOR₆ |
| aryl | COOH | aryl | aryl | (CH₂)ₙOR₆ |
| aryl | COOH | aryl | (CH₂)ₙCOOR₆ | H |
| aryl | COOH | aryl | (CH₂)ₙCOOR₆ | alkyl |
| aryl | COOH | aryl | (CH₂)ₙCOOR₆ | aryl |
| aryl | COOH | aryl | (CH₂)ₙCOOR₆ | (CH₂)ₙCOOR₆ |
| aryl | COOH | aryl | (CH₂)ₙCOOR₆ | (CH₂)ₙOR₆ |
| aryl | COOH | aryl | (CH₂)ₙOR₆ | H |
| aryl | COOH | aryl | (CH₂)ₙOR₆ | alkyl |
| aryl | COOH | aryl | (CH₂)ₙOR₆ | aryl |
| aryl | COOH | aryl | (CH₂)ₙOR₆ | (CH₂)ₙCOOR₆ |
| aryl | COOH | aryl | (CH₂)ₙOR₆ | (CH₂)ₙOR₆ |
| aryl | NHR₆ | H | H | H |
| aryl | NHR₆ | H | H | alkyl |
| aryl | NHR₆ | H | H | aryl |
| aryl | NHR₆ | H | H | (CH₂)ₙCOOR₆ |
| aryl | NHR₆ | H | H | (CH₂)ₙOR₆ |
| aryl | NHR₆ | H | alkyl | H |
| aryl | NHR₆ | H | alkyl | alkyl |
| aryl | NHR₆ | H | alkyl | aryl |
| aryl | NHR₆ | H | alkyl | (CH₂)ₙCOOR₆ |
| aryl | NHR₆ | H | alkyl | (CH₂)ₙOR₆ |
| aryl | NHR₆ | H | aryl | H |
| aryl | NHR₆ | H | aryl | alkyl |
| aryl | NHR₆ | H | aryl | aryl |
| aryl | NHR₆ | H | aryl | (CH₂)ₙCOOR₆ |
| aryl | NHR₆ | H | aryl | (CH₂)ₙOR₆ |
| aryl | NHR₆ | H | (CH₂)ₙCOOR₆ | H |
| aryl | NHR₆ | H | (CH₂)ₙCOOR₆ | alkyl |
| aryl | NHR₆ | H | (CH₂)ₙCOOR₆ | aryl |
| aryl | NHR₆ | H | (CH₂)ₙCOOR₆ | (CH₂)ₙCOOR₆ |
| aryl | NHR₆ | H | (CH₂)ₙCOOR₆ | (CH₂)ₙOR₆ |
| aryl | NHR₆ | H | (CH₂)ₙOR₆ | H |
| aryl | NHR₆ | H | (CH₂)ₙOR₆ | alkyl |
| aryl | NHR₆ | H | (CH₂)ₙOR₆ | aryl |
| aryl | NHR₆ | H | (CH₂)ₙOR₆ | (CH₂)ₙCOOR₆ |
| aryl | NHR₆ | H | (CH₂)ₙOR₆ | (CH₂)ₙOR₆ |
| aryl | NHR₆ | alkyl | H | H |
| aryl | NHR₆ | alkyl | H | alkyl |
| aryl | NHR₆ | alkyl | H | aryl |
| aryl | NHR₆ | alkyl | H | (CH₂)ₙCOOR₆ |
| aryl | NHR₆ | alkyl | H | (CH₂)ₙOR₆ |
| aryl | NHR₆ | alkyl | alkyl | H |
| aryl | NHR₆ | alkyl | alkyl | alkyl |
| aryl | NHR₆ | alkyl | alkyl | aryl |
| aryl | NHR₆ | alkyl | alkyl | (CH₂)ₙCOOR₆ |
| aryl | NHR₆ | alkyl | alkyl | (CH₂)ₙOR₆ |
| aryl | NHR₆ | alkyl | aryl | H |
| aryl | NHR₆ | alkyl | aryl | alkyl |
| aryl | NHR₆ | alkyl | aryl | aryl |
| aryl | NHR₆ | alkyl | aryl | (CH₂)ₙCOOR₆ |
| aryl | NHR₆ | alkyl | aryl | (CH₂)ₙOR₆ |
| aryl | NHR₆ | alkyl | (CH₂)ₙCOOR₆ | H |
| aryl | NHR₆ | alkyl | (CH₂)ₙCOOR₆ | alkyl |
| aryl | NHR₆ | alkyl | (CH₂)ₙCOOR₆ | aryl |
| aryl | NHR₆ | alkyl | (CH₂)ₙCOOR₆ | (CH₂)ₙCOOR₆ |
| aryl | NHR₆ | alkyl | (CH₂)ₙCOOR₆ | (CH₂)ₙOR₆ |
| aryl | NHR₆ | alkyl | (CH₂)ₙOR₆ | H |
| aryl | NHR₆ | alkyl | (CH₂)ₙOR₆ | alkyl |
| aryl | NHR₆ | alkyl | (CH₂)ₙOR₆ | aryl |
| aryl | NHR₆ | alkyl | (CH₂)ₙOR₆ | (CH₂)ₙCOOR₆ |
| aryl | NHR₆ | alkyl | (CH₂)ₙOR₆ | (CH₂)ₙOR₆ |
| aryl | NHR₆ | aryl | H | H |
| aryl | NHR₆ | aryl | H | alkyl |
| aryl | NHR₆ | aryl | H | aryl |
| aryl | NHR₆ | aryl | H | (CH₂)ₙCOOR₆ |
| aryl | NHR₆ | aryl | H | (CH₂)ₙOR₆ |
| aryl | NHR₆ | aryl | alkyl | H |
| aryl | NHR₆ | aryl | alkyl | alkyl |
| aryl | NHR₆ | aryl | alkyl | aryl |
| aryl | NHR₆ | aryl | alkyl | (CH₂)ₙCOOR₆ |
| aryl | NHR₆ | aryl | alkyl | (CH₂)ₙOR₆ |
| aryl | NHR₆ | aryl | aryl | H |
| aryl | NHR₆ | aryl | aryl | alkyl |
| aryl | NHR₆ | aryl | aryl | aryl |
| aryl | NHR₆ | aryl | aryl | (CH₂)ₙCOOR₆ |
| aryl | NHR₆ | aryl | aryl | (CH₂)ₙOR₆ |
| aryl | NHR₆ | aryl | (CH₂)ₙCOOR₆ | H |
| aryl | NHR₆ | aryl | (CH₂)ₙCOOR₆ | alkyl |
| aryl | NHR₆ | aryl | (CH₂)ₙCOOR₆ | aryl |
| aryl | NHR₆ | aryl | (CH₂)ₙCOOR₆ | (CH₂)ₙCOOR₆ |
| aryl | NHR₆ | aryl | (CH₂)ₙCOOR₆ | (CH₂)ₙOR₆ |
| aryl | NHR₆ | aryl | (CH₂)ₙOR₆ | H |
| aryl | NHR₆ | aryl | (CH₂)ₙOR₆ | alkyl |
| aryl | NHR₆ | aryl | (CH₂)ₙOR₆ | aryl |
| aryl | NHR₆ | aryl | (CH₂)ₙOR₆ | (CH₂)ₙCOOR₆ |
| aryl | NHR₆ | aryl | (CH₂)ₙOR₆ | (CH₂)ₙOR₆ |
| aryl | (CH₂)ₙCOOR₆ | H | H | H |
| aryl | (CH₂)ₙCOOR₆ | H | H | alkyl |
| aryl | (CH₂)ₙCOOR₆ | H | H | aryl |
| aryl | (CH₂)ₙCOOR₆ | H | H | (CH₂)ₙCOOR₆ |
| aryl | (CH₂)ₙCOOR₆ | H | H | (CH₂)ₙOR₆ |
| aryl | (CH₂)ₙCOOR₆ | H | alkyl | H |
| aryl | (CH₂)ₙCOOR₆ | H | alkyl | alkyl |
| aryl | (CH₂)ₙCOOR₆ | H | alkyl | aryl |
| aryl | (CH₂)ₙCOOR₆ | H | alkyl | (CH₂)ₙCOOR₆ |
| aryl | (CH₂)ₙCOOR₆ | H | alkyl | (CH₂)ₙOR₆ |
| aryl | (CH₂)ₙCOOR₆ | H | aryl | H |
| aryl | (CH₂)ₙCOOR₆ | H | aryl | alkyl |
| aryl | (CH₂)ₙCOOR₆ | H | aryl | aryl |
| aryl | (CH₂)ₙCOOR₆ | H | aryl | (CH₂)ₙCOOR₆ |
| aryl | (CH₂)ₙCOOR₆ | H | aryl | (CH₂)ₙOR₆ |
| aryl | (CH₂)ₙCOOR₆ | H | (CH₂)ₙCOOR₆ | H |
| aryl | (CH₂)ₙCOOR₆ | H | (CH₂)ₙCOOR₆ | alkyl |
| aryl | (CH₂)ₙCOOR₆ | H | (CH₂)ₙCOOR₆ | aryl |
| aryl | (CH₂)ₙCOOR₆ | H | (CH₂)ₙCOOR₆ | (CH₂)ₙCOOR₆ |
| aryl | (CH₂)ₙCOOR₆ | H | (CH₂)ₙCOOR₆ | (CH₂)ₙOR₆ |
| aryl | (CH₂)ₙCOOR₆ | H | (CH₂)ₙOR₆ | H |
| aryl | (CH₂)ₙCOOR₆ | H | (CH₂)ₙOR₆ | alkyl |
| aryl | (CH₂)ₙCOOR₆ | H | (CH₂)ₙOR₆ | aryl |
| aryl | (CH₂)ₙCOOR₆ | H | (CH₂)ₙOR₆ | (CH₂)ₙCOOR₆ |
| aryl | (CH₂)ₙCOOR₆ | H | (CH₂)ₙOR₆ | (CH₂)ₙOR₆ |
| aryl | (CH₂)ₙCOOR₆ | alkyl | H | H |
| aryl | (CH₂)ₙCOOR₆ | alkyl | H | alkyl |
| aryl | (CH₂)ₙCOOR₆ | alkyl | H | aryl |
| aryl | (CH₂)ₙCOOR₆ | alkyl | H | (CH₂)ₙCOOR₆ |
| aryl | (CH₂)ₙCOOR₆ | alkyl | H | (CH₂)ₙOR₆ |
| aryl | (CH₂)ₙCOOR₆ | alkyl | alkyl | H |
| aryl | (CH₂)ₙCOOR₆ | alkyl | alkyl | alkyl |
| aryl | (CH₂)ₙCOOR₆ | alkyl | alkyl | aryl |
| aryl | (CH₂)ₙCOOR₆ | alkyl | alkyl | (CH₂)ₙCOOR₆ |
| aryl | (CH₂)ₙCOOR₆ | alkyl | alkyl | (CH₂)ₙOR₆ |
| aryl | (CH₂)ₙCOOR₆ | alkyl | aryl | H |
| aryl | (CH₂)ₙCOOR₆ | alkyl | aryl | alkyl |
| aryl | (CH₂)ₙCOOR₆ | alkyl | aryl | aryl |
| aryl | (CH₂)ₙCOOR₆ | alkyl | aryl | (CH₂)ₙCOOR₆ |
| aryl | (CH₂)ₙCOOR₆ | alkyl | aryl | (CH₂)ₙOR₆ |
| aryl | (CH₂)ₙCOOR₆ | alkyl | (CH₂)ₙCOOR₆ | H |
| aryl | (CH₂)ₙCOOR₆ | alkyl | (CH₂)ₙCOOR₆ | alkyl |
| aryl | (CH₂)ₙCOOR₆ | alkyl | (CH₂)ₙCOOR₆ | aryl |
| aryl | (CH₂)ₙCOOR₆ | alkyl | (CH₂)ₙCOOR₆ | (CH₂)ₙCOOR₆ |
| aryl | (CH₂)ₙCOOR₆ | alkyl | (CH₂)ₙCOOR₆ | (CH₂)ₙOR₆ |
| aryl | (CH₂)ₙCOOR₆ | alkyl | (CH₂)ₙOR₆ | H |
| aryl | (CH₂)ₙCOOR₆ | alkyl | (CH₂)ₙOR₆ | alkyl |
| aryl | (CH₂)ₙCOOR₆ | alkyl | (CH₂)ₙOR₆ | aryl |
| aryl | (CH₂)ₙCOOR₆ | alkyl | (CH₂)ₙOR₆ | (CH₂)ₙCOOR₆ |
| aryl | (CH₂)ₙCOOR₆ | alkyl | (CH₂)ₙOR₆ | (CH₂)ₙOR₆ |
| aryl | (CH₂)ₙCOOR₆ | aryl | H | H |
| aryl | (CH₂)ₙCOOR₆ | aryl | H | alkyl |
| aryl | (CH₂)ₙCOOR₆ | aryl | H | aryl |
| aryl | (CH₂)ₙCOOR₆ | aryl | H | (CH₂)ₙCOOR₆ |
| aryl | (CH₂)ₙCOOR₆ | aryl | H | (CH₂)ₙOR₆ |
| aryl | (CH₂)ₙCOOR₆ | aryl | alkyl | H |
| aryl | (CH₂)ₙCOOR₆ | aryl | alkyl | alkyl |
| aryl | (CH₂)ₙCOOR₆ | aryl | alkyl | aryl |
| aryl | (CH₂)ₙCOOR₆ | aryl | alkyl | (CH₂)ₙCOOR₆ |
| aryl | (CH₂)ₙCOOR₆ | aryl | alkyl | (CH₂)ₙOR₆ |
| aryl | (CH₂)ₙCOOR₆ | aryl | aryl | H |
| aryl | (CH₂)ₙCOOR₆ | aryl | aryl | alkyl |
| aryl | (CH₂)ₙCOOR₆ | aryl | aryl | aryl |
| aryl | (CH₂)ₙCOOR₆ | aryl | aryl | (CH₂)ₙCOOR₆ |
| aryl | (CH₂)ₙCOOR₆ | aryl | aryl | (CH₂)ₙOR₆ |
| aryl | (CH₂)ₙCOOR₆ | aryl | (CH₂)ₙCOOR₆ | H |
| aryl | (CH₂)ₙCOOR₆ | aryl | (CH₂)ₙCOOR₆ | alkyl |
| aryl | (CH₂)ₙCOOR₆ | aryl | (CH₂)ₙCOOR₆ | aryl |
| aryl | (CH₂)ₙCOOR₆ | aryl | (CH₂)ₙCOOR₆ | (CH₂)ₙCOOR₆ |
| aryl | (CH₂)ₙCOOR₆ | aryl | (CH₂)ₙCOOR₆ | (CH₂)ₙOR₆ |
| aryl | (CH₂)ₙCOOR₆ | aryl | (CH₂)ₙOR₆ | H |
| aryl | (CH₂)ₙCOOR₆ | aryl | (CH₂)ₙOR₆ | alkyl |
| aryl | (CH₂)ₙCOOR₆ | aryl | (CH₂)ₙOR₆ | aryl |
| aryl | (CH₂)ₙCOOR₆ | aryl | (CH₂)ₙOR₆ | (CH₂)ₙCOOR₆ |
| aryl | (CH₂)ₙCOOR₆ | aryl | (CH₂)ₙOR₆ | (CH₂)ₙOR₆ |

The invention also relates to a method for the preparation of radiolabeled biomolecules comprising:
a) contacting a chelating agent of the invention with a carbonyl moiety of the formula [M(CO)₃]⁺, wherein M is rhenium (Re) or technetium (Tc), under conditions for forming a chelator-carbonyl complex; and
b) contacting the complex with a biomolecule for obtaining a radiolabeled biomolecule. This method is in particular useful for labeling biomolecules that are sensitive to temperature and extreme pH.

This method can for example be performed with a kit, comprising a first vial with the chelating agent of the invention, optionally a first reaction vial for contacting the chelating agent with the carbonyl moiety, a second vial with the biomolecule and optionally a second reaction vial for reacting the biomolecule with the chelator-carbonyl complex obtained in the first step of the reaction.

In an alternative embodiment the invention provides a method for the preparation of radiolabeled biomolecules comprising:
a) contacting a chelating agent of the invention with a biomolecule for obtaining a chelator-biomolecule.; and
b) contacting the chelator-biomolecule with a carbonyl moiety of the formula [M(CO)₃]⁺, wherein M is rhenium (Re) or technetium (Tc) under conditions for forming a radiolabeled biomolecule.

A kit for performing this method comprises for example a first vial with the chelating agent of the invention, optionally a first reaction vial for reacting the chelating agent with the biomolecule, a second vial with the carbonyl moiety and optionally a second reaction vial for reacting the chelator-biomolecule obtained in the first step of the reaction with the carbonyl.

The invention will be further illustrated in the example that follows.

### EXAMPLE

### Introduction

The bifunctional pyrazolyl-polyamines, pyrazolyl-polythioether, pyrazolyl amino-thioether ligands, pyrazolyl-aminophosphines and pyrazolyl-thioetherphosphines contain different donor atom sets to stabilize the metal and have different functional groups in different positions to which seeking molecules such as, for example, monoclonal antibodies, peptides, oligonucleotides and glycoproteins, can be coupled. They can also have different substituents and alkyl chains in different positions of the backbone for tuning the physico-chemical properties of the molecules.

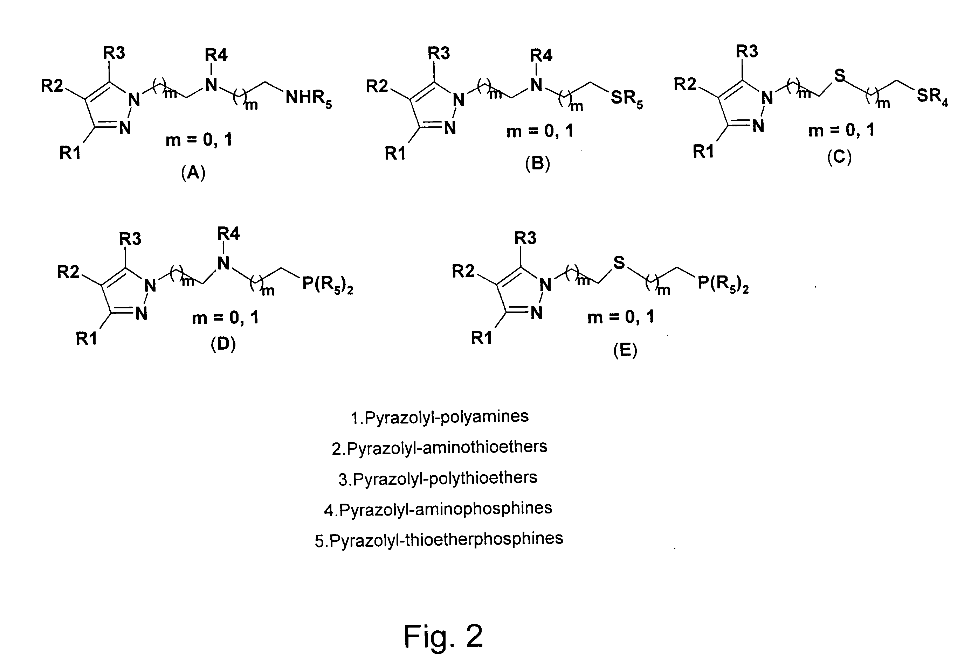

A general overview is given in Figure 1, showing possible combinations for metal fragments of the type [M(CO)₃]⁺ (M = Re, Tc, Mn). The five different types of bifunctional tridentate pyrazolyl-containing ligands, which are subject of this invention are depicted schematically in Figure 2.

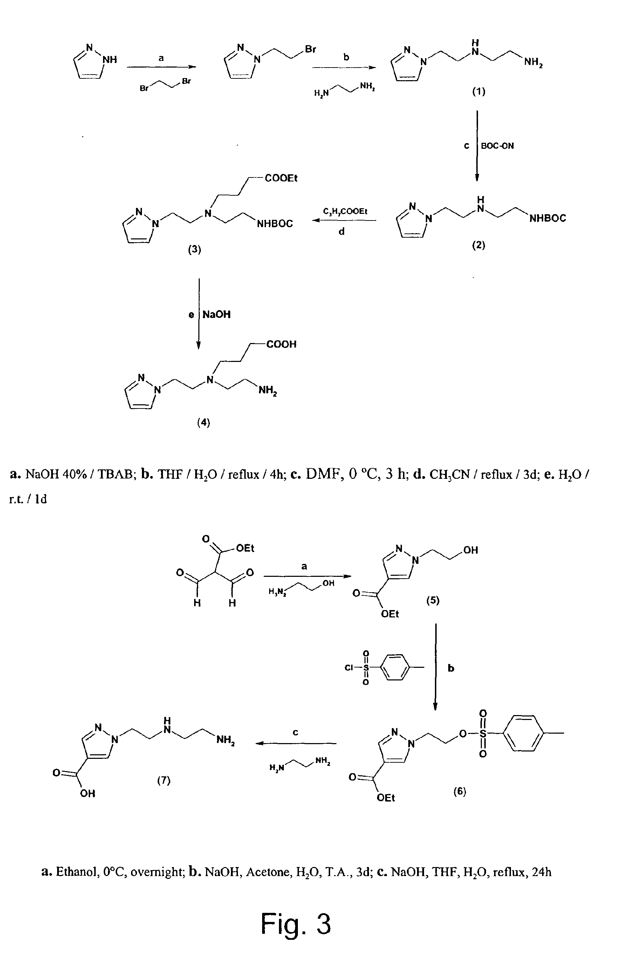

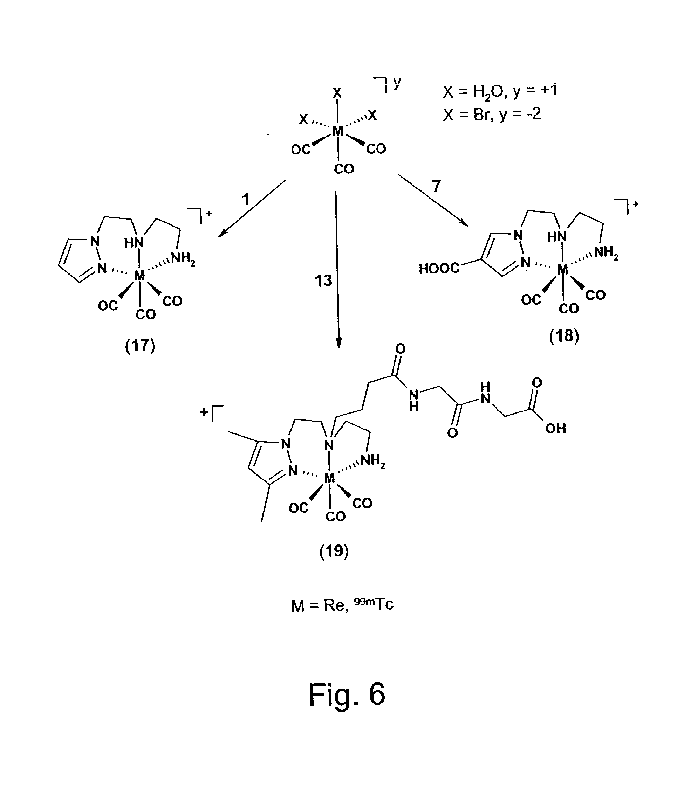

The present invention will be further illustrated in the Figures 3-6, which are solely intended to clarify the invention. This family of ligands led to thermodynamically stable complexes and the versatility of the backbone is an important factor for tuning the physico-chemical properties of the compounds and obviously its pharmacokinetics. In Figure 6, some of the Re and Tc complexes referred as examples are schematically represented.

### Materials and methods

### 1. Synthesis of 2-[2-(pyrazol-1-yl)ethylimino]ethylamine (pz(CH₂)₂NH(CH₂)₂NH₂) (1) (see Figure 3)

A solution of **1-(2-bromoethyl)pyrazole** [6d] (12 mmol) in tetrahydrofuran was added dropwise to a solution of ethylenediamine (0.24 mol) in water. The mixture was refluxed for 4 hours. The THF was removed under vacuum and the water phase was washed with dichloromethane. After drying under vacuum resulted a yellow oil which was formulated as pz(CH₂)₂NH(CH₂)₂NH₂ (1).
Yield: 50%
¹H-NMR (D₂O): 7.53 (d, H(3)pz, 1H); 7.45 (d, H(5)pz, 1H) ; 6.23 (t, H(4)pz, 1H); 4.14 (t, CH2, 2H); 2.91 (t, CH₂, 2H); 2.77
(t, CH₂, 2H); 2.62 (t, CH₂, 2H).

### 2. Synthesis pz(CH₂)₂N[(CH₂)₃COOH](CH₂)₂NH₂ (4) (see Figure 3)

### 2.1. BOC-ON protection

A solution of **1** (1.1 g; 7 mmol) in DMF (20 ml) was cooled to 0°C and a solution of BOC-ON (1,7 g; 7 mmol) in DMF (20 ml) was added dropwise. The reaction mixture was stirred for 3 hours at 0°C. The solvent was removed under vacuum and the solid residue was dissolved in water and washed with chloroform 3 times, yielding **2** as an oil. Yield: 68%. ¹H-NMR (D₂O): 7.55 (d, H(3)pz, 1H); 7.47 (d, H(5)pz, 1H); 6.24 (t, H(4)pz, 1H); 4.38 (t, CH₂, 2H); 3.40 (t, CH₂, 2H); 3.20 (t, CH₂, 2H); 2.99 (t, CH₂, 2H); 1.25 (s, CH₃, 9H).

### 2.2. Alkylation with ethyl 4-bromobutyrate, hydrolysis and deprotection

Compound **2** (757 mg; 3 mmol) was dissolved in 10 ml of acetonitrile. Potassium carbonate (829 mg; 6 mmol) and a catalytic amount of potassium iodide were added to the solution, and ethyl 4-bromobutyrate (858 ml, 16 mmol) was added dropwise. After refluxing for 3 days, the supernatant was separated by filtration and vacuum dried leading to **3**. This compound (733 mg, 2 mmol) was dissolved in an aqueous solution of NaOH (800 mg, 20 mmol) and reacted for one day at room temperature. The solution was then neutralized with HCl 1N and vacuum dried. The solid residue was dissolved in methanol, the precipitating salts were filtered off, and the solvent was removed under vacuum, yielding a yellow/brown oil formulated as **4**. Yield: (50%).
¹H-NMR (D₂O): 7. 78 (d, H(3)pz, 1H); 7.64 (d, H(5)pz, 1H); 6.42 (t, H(4)pz, 1H); 4.36 (t, CH₂, 2H); 3.10 (t, CH₂, 2H); 3.02 (t, CH₂, 2H); 2.86 (t, CH₂, 2H); 2.64 (t, CH₂, 2H); 2.15 (t, CH₂, 2H); 1.68 (q, CH₂, 2H).

### 3. Synthesis (4-carboxylic)pz(CH₂)₂NH(CH₂)₂NH₂ (7) (see Figure 3)

### 3.1. Ethyl N-2-hydroxyethyl-4-pyrazolecarboxylate (5)

Compound **5** was prepared using the classical approach for preparing pyrazoles [7]. Ethyl 2-formyl-3-oxopropionate (2.80 g; 20 mmol) was dissolved in 20 ml of ethanol and cooled to 0°C. 2-Hydroxyethylhydrazine (1.44 g; 20 mmol) was dissolved in 100 ml of ethanol and was added dropwise to the solution of ethyl 2-formyl-3-oxopropionate. The reaction mixture was left overnight at room temperature. The solvent was vacuum removed yielding a yellow oil. Yield: 95%
¹H-NMR (CDCl₃) : 7.93 (s, H(3)pz, 1H); 7.91 (s, H(5)pz, 1H); 4.30-4.22 (m, CH₂+OCH₂, 5H); 3.99 (t, CH₂, 2H); 1.30 (t, CH₃, 3H).

### 3.2. Ethyl N-(2-p-toluenesulfonylethyl)-4-pyrazole-carboxylate (6)

Ethyl N-2-hydroxyethyl-4-pyrazolecarboxylate **(5)** (2.76 g, 15 mmol) and p-toluenesulfonylchloride (2.85 g, 15 mmol) were suspended in a solution of acetone (15 ml) and water (15 ml) and cooled to 0°C. A solution of NaOH (0.6 g, 15 mmol) in water (10 ml) was added dropwise for 15 min. The mixture was then allowed to reach the room temperature and was vigorously stirred overnight. The acetone was evaporated and the aqueous solution was extracted 3 times with chloroform, yielding a yellow oil. Yield: 60%
¹H-NMR (CDCl₃): 7.82 (s, H(3)pz, 1H) ; 7.76 (s, H(5)pz, 1H); 7.61 (d, H(ph), 2H); 7.26 (d, H(ph), 2H); 4.35 (q, OCH₂, 2H); 4.24 (t, CH₂, 2H); 2.15 (s, CH₃, 3H) 1.33 (t, CH₂, 2H).

Compound **7** was prepared as follows. Ethylenediamine (16 ml; 0.24 mol) was dissolved in a solution of NaOH (9.6 g; 0.24 mol) in water (20 ml). A solution of Ethyl N-(2-p-toluenesulfonylethyl)-4-pyrazolecarboxylate **(6)** (4.06 g; 12 mmol) in THF (10 ml) was added dropwise to the ethylenediamine solution. The reaction mixture was refluxed for 24 hours. After that, the solvent was vacuum removed and the product was purified by column chromatography in silica-gel (eluent:methanol-NH₃/methanol (50:50)), yielding a dark yellow solid. Yield: 50%.
¹H-NMR (D₂O): δ 7.80 (s, H(3)pz, 1H); 7.64 (s, H(5)pz, 1H); 4.27 (t, CH₂, 2H); 3.24 (t, CH₂, 2H); 3.11-3.00 (m, 2CH₂, 4H). IV (KBr) (ν/cm⁻¹): 1690 (C=O).

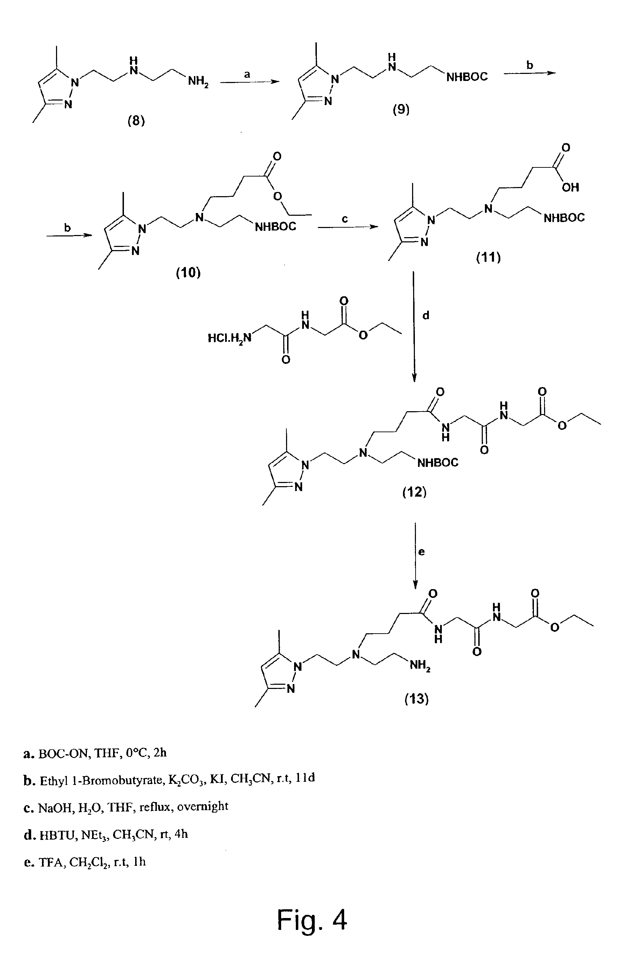

### 4. Synthesis of 3,5-Mepz(CH₂)₂N[(CH₂)₃GlyGlyOEt)](CH₂)₂NH₂ (13) (Figure 4)

### 4.1. BOC-ON protection (9)

Compound **8** (3.41 g, 18.71 mmol) [4c] was dissolved in THF (25 mL) and cooled to a temperature between -10°C and 0°C. BOC-ON (4.60 g, 18.71 mmol) in THF (20 ml) was added dropwise and the reaction mixture was stirred for 2 h at 0°C, resulting in the complete conversion of **8** as monitored by TLC (*R*_{*f*} = 0.5, 100% MeOH). The reaction mixture was then warmed to room temperature and partitioned between a saturated aqueous Na₂CO₃ solution and dichloromethane. The organic layer was separated, dried over anhydrous MgSO₄, filtered, and concentrated under reduced pressure to afford the product **9** in quantitative yield (by ¹H-NMR), as a highly viscous colorless oil. This product was used in the next step without further purification.
¹H-NMR (CDCl₃): δ 5.76 (s, pyrazol, 1H), 5.08 (s br., NH, 1H), 4.04 (t, CH₂, 2H), 3.18 (m, CH₂, 2H), 2.99 (t, CH₂, 2H), 2.72 (t, CH₂, 2H), 2.18 (s, CH₃, 3H), 2.20 (s, CH₃, 3H), 1.40 (s, C (CH₃)₃, 9H).

### 4.2. Synthesis of 3,5-Me₂pz(CH₂)₂N[(CH₂)₃COOH](CH₂)₂NHBOC (11)

To a stirred solution of the crude product **9** (1.02 g) in CH₃CN (15 mL), ethyl 4-bromobutyrate (1.4 g, 7.20 mmol), K₂CO₃ (1.00 g, 7.20 mmol) and a catalytic amount of KI were added. The obtained suspension was allowed to react under vigorous stirring for 11 days, being the reaction monitored by TLC (*R*_{*f*} product = 0.4, 10% MeOH/CH₂Cl₂). After elimination of the white solids in suspension by filtration, the solvent was evaporated in vacuum and a pale-yellow viscous oil was obtained. The crude product was chromatographed on an appropriate column of silica gel with 75-100% ethyl acetate/hexane (gradient) to afford **10** as a pale-yellow viscous oil, which solidifies on standing for several days at room temperature. Yield: 0.73 g (51% yield).

A solution of **10** (4.6 g, 11.60 mmol) in THF (190 mL) and aqueous NaOH (8.3 mL of a 14 N NaOH solution, 116.0 mmol) was refluxed for 8 h. The reaction was monitored by TLC (*R*_{*f*} product = 0.2, 10% MeOH/CH₂Cl₂). After neutralization with HCl 4N (pH 6-7), the THF/H₂O solution was evaporated to dryness under reduced pressure. The crude product was chromatographed on an appropriate column of silica gel with 10-50% MeOH/CHCl₃ (gradient) to afford **11** as an highly viscous colorless oil, which crystallizes on standing after several days. Yield: 2.82 g (66%).

Compound **10:** ¹H-NMR (CDCl₃): δ 5.75 (s, pyrazol, 1H), 4.09 (q, CH₂, 2H), 3.98 (s br., CH₂, 2H), 3.08 (s br., CH₂, 2H), 2.78 (s br., CH₂, 2H), 2.45-2.51 (m, CH₂, 4H), 2.23 (s, CH₃, 3H), 2.18 (m, CH₃ CH₂, 5H), 1.63 (s br., CH₂, 2H), 1.41 (s, C(CH₃)₃, 9H), 1.23 (t, CH₃, 3H).

Compound 11: ¹H-NMR (CDCl₃): δ 5.81 (s, pyrazol, 1H), 4.93(s br., NH, 1H) 4.12 (t br., CH₂, 2H), 3.04 (q br., CH₂, 2H), 2.86 (t br., CH₂, 2H), 2.58-2.64 (m, CH₂, 4H), 2.42 (t, CH₂, 2H), 2.24 (s, CH₃, 3H), 2.19 (s, CH₃, 3H), 1.79 (m, CH₂, 2H), 1.40 (s, C(CH₃)₃, 9H).

Compound 3,5-Me₂pz(CH₂)₂N[(CH₂)₃CONHGlyGlyOEt](CH₂)₂NH₂ **(13)** was prepared as follows (see Figure 4).

To a solution of **11** (1.51 g, 4.09 mmol) in CH₃CN (48 mL) were added GlyGly ethyl ester hydrochloride (0.57 g, 4.09 mmol), triethylamine (1.24g, 12.27 mmol), and HBTU (1.55 g, 4.09 mmol). The reaction mixture was stirred 20 h at room temperature under nitrogen. The reaction was monitored by TLC (*R*_{*f*} product = 0.8, 20% MeOH/CH₂Cl₂). The solvent was evaporated and the crude product obtained was purified by chromatography on an appropriate silica gel column with 3-5% MeOH/CHCl₃ (gradient) to afford **12** as a viscous colorless oil. Yield: 1.23 g (59%).

A solution of 3,5-Me₂pz(CH₂)₂N[(CH₂)₃CONHGlyGlyOEt] (CH₂)₂NHBOC (**12**) (1.23 g, 2.41 mmol) in CH₂Cl₂/TFA (25 mL / 4.1 mL) was allowed to react for 2 h. The reaction was monitored by TLC (*R*_{*f*} = 0.4, 20% MeOH/CH₂Cl₂). The solvent and the TFA were evaporated under reduced pressure and a highly viscous pale-yellow oil was obtained. This oil was dissolved in water, neutralized with NaOH 1N (pH 7-8) and the solvent evaporated to dryness. TLC: *R*_{*f*} = 0.2, 20% MeOH/CH₂Cl₂ The compound was further purified by chromatography on an appropriate silica gel column with 20-40% MeOH/CHCl₃ (gradient) to afford **13** as a viscous colorless oil. Yield: 0.97 g (98 %).

Compound **12:** ¹H-NMR (CDCl₃): δ 8.66 (s br., NH, 1H), 7.00 (s br., NH, 1H), 5.80 (s, pyrazol, 1H), 4.91 (s br., NH, 1H) 4.15 (q., CH₂, 2H), 4.04 (s br., CH₂, 2H), 3.97 (d, CH₂, 2H), 3.90 (d, CH₂, 2H), 2.89 (s br., CH₂, 2H), 2.69 (s br., CH₂, 2H), 2.51 (s br., CH₂, 2H), 2.39 (s br., CH₂, 2H), 2.30 (s br., CH₂, 2H), 2.20 (s, CH₃, 3H), 2.18 (s, CH₃, 3H), 1.74 (s br., CH₂, 2H), 1.38 (s, C(CH₃)₃, 9H), 1.23 (t, CH₃, 3H).

Compound **13:** ¹H-NMR (CD3OD): δ 5.84 (s, pyrazol, 1H), 4.17 (q, CH₂, 2H), 4.06 (t, CH₂, 2H), 3.91 (d, CH₂, 4H), 2.97 (t, CH₂, 2H), 2.71-2.80 (m, CH₂, 4H), 2.51 (t, CH₂, 2H), 2.25 (s, CH₃, 3H), 2.15 (s, CH₃, 3H), 2.12 (t, CH₂, 2H), 1.66 (m, CH₂, 2H), 1.25 (t, CH₃, 3H).

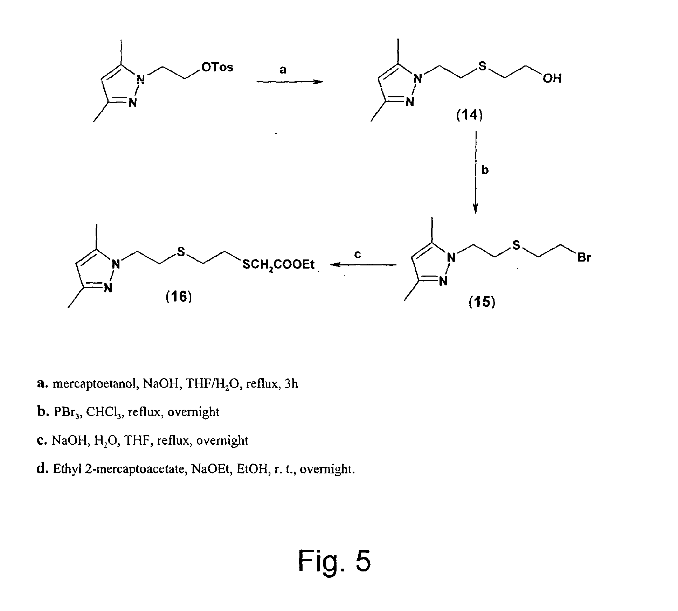

### 5. Synthesis of 3,5-Mepz(CH₂)₂S(CH₂)₂S(CH₂)COOEt (16) (Figure 5)

### 5.1 Synthesis of 3,5-Mepz(CH₂)₂S(CH₂)₂OH (14)

0.70 ml (10 mmol) of HSCH₂CH₂OH were mixed with 0.40 g (10 mmol) of NaOH, in water, and the solution was refluxed for 5 min. To this solution, 2.78 g (10 mmol) of N-(2-p-toluenesulfonylethyl)-3,5-dimethylpyrazole dissolved in tetrahydrofuran (THF) were added dropwise at room temperature, followed by gentle reflux for 3 hr. The mixture was extracted with chloroform from which, after drying under vacuum, were recovered 1.62g of **14** as a yellow oil (8.10 mmol, 81%).

Compound **14:** ¹H-NMR (CDCl₃): 5.67 (s, pz-H, 1H); 4.39 (s, OH, 1H); 4.03 (t, CH₂, 2H); 3.60 (t, CH₂, 2H); 2.83 (t, CH₂, 2H); 2.50 (t, CH₂, 2H); 2.14 (s, CH₃, 3H); 2.09 (s, CH₃, 3H).

### 5.2 Synthesis of 3,5-Mepz(CH₂)₂S(CH₂)₂Br (15)

0.19 ml (2 mmol) of PBr₃ were added to **14** (0.40 g, 2 mmol) dissolved in chloroform, and the resulting solution was refluxed for 24 hours under N₂. The mixture was treated with 20 ml of 10% NaHCO₃ solution. The organic phase was separated and chloroform removed under vacuum, yielding 0.329 g of **15** as a yellow oil (1.25 mmol, 63% ).
¹H-NMR (CD_{C13}): 5.82 (s, pz-H, 1H) ; 4.15 (t, CH₂, 2H); 3.36 (t, CH₂, 2H); 3.00 (t, CH₂, 2H); 2.70 (t, CH₂, 2H); 2.26 (s, CH₃, 3H); 2.23 (s, CH₃, 3H).

Under N₂, dry ethanol was added to metallic sodium (0.15 g, 4.56 mmol), and the mixture was stirred at room temperature until complete conversion to sodium ethoxide. To this mixture an ethanolic solution of ethyl 2-mercaptoacetate (0.50 ml, 4.56 mmol)was added dropwise, followed by addition of 1.20 g (4.56 mmol) of 3,5-Mepz(CH₂)₂S(CH₂)₂Br (**15**) in ethanol. The reaction mixture was stirring overnight at room temperature. After this time, the solvent was removed under vacuum and the resulting oil was dissolved in chloroform. After washing with water, the organic phase was dried under vacuum yielding 1.00 g of **16** as a yellow oil (3.3 mmol, 72.4%).

Compound **16**: ¹H-NMR (CDCl₃): 5.82 (s, pz-H, 1H); 4.14 (m, CH_{2'} CH₂-COO, 4H); 3.25 (s, CH₂, 2H); 2.92 (t, CH₂, 2H); 2.75 (t, CH₂, 2H); 2.57 (t, CH₂, 2H); 2.2 (s, CH₃, 3H); 2.16 (s, CH₃, 3H); 1.25 (t, CH₃, 3H).

### 6. Re and Tc compounds (see Figure 6)

### 6.1. Synthesis of [Re(CO)₃(κ³-pz(CH₂)₂NH(CH₂)₂NH₂)]Br (17a)

100 mg (0.130 mmol) of (NEt₄)₂[ReBr₃(CO)₃] were mixed with 20 mg (0.130 mmol) of the compound 1 (pz(CH₂)₂NH(CH₂)₂NH₂) in water, and the solution was refluxed for 2 hours. The volume was then reduced under vacuum, and the mixture was left at 4°C until a white solid precipitated. Yield: >90% by ¹H-NMR
¹H-NMR (D₂O): 7.82 (d, H(3)pz, 1H); 7.76 (d, H(5)pz, 1H); 6.54 (s br, NH, 1H 6.39 (t, H(4)pz, 1H); 4.86 (s, largo, NH₂, 1H); 4.43 (m, CH_{2,} 1H); 4.16 (m, CH_{2,} 1H); 3.94 (s, largo, NH₂, 1H); 3.50 (m, CH₂, 1H); 2.87 (m, CH₂, 1H); 2.71 (m, CH₂, 2H); 2.48 (m, CH₂, 1H); 2.08 (m, CH₂, 1H).

### 6.2. Synthesis of [^{99m}Tc(CO)₃(κ³-pz(CH₂)₂NH(CH₂)₂NH₂)]+ (17b)

100 µl of a solution of compound 1 **(pz(CH**_{**2**}**)**_{**2**}**NH(CH**_{**2**}**)**_{**2**}**NH**_{**2**}**)** 10⁻⁴ M was added to 1 ml of a solution of [^{99m}Tc(OH)₃(CO)₃]+ (1-2 mCi) in phosphate buffer. The solution was incubated for 30 min at 100°C and then analyzed by HPLC. The radiochemical purity was >90%.

### 6.3. Synthesis of [Re(CO)₃(κ³-(4-carboxylic acid)pz(CH₂)₂NH (CH₂)₂NH₂)]Br (18a)

100 mg (0.130 mmol) of (NEt₄)₂[ReBr3(CO)₃] were mixed with 26 mg (0.130 mmol) of compound **7**, in water, and the solution was refluxed for 2 hours. The volume was then reduced under vacuum, and the mixture was left at 4°C until a white solid precipitated.
Yield: >90% by ¹H-NMR
¹H-NMR (D₂O): δ 8.22 (s, H(3)pz, 1H); 8.20 (s, H(5)pz, 1H); 6.62 (s, largo, NH, 1H); 4.94 (s, largo, NH₂, 1H); 4.43 (m, CH_{2,} 1H); 4.25 (m, CH₂, 1H); 4.05 (s, largo, NH₂. 1H); 3.52 (m, CH₂, 1H); 2.92 (m, CH₂, 1H); 2.76 (m, CH₂, 2H); 2.53 (m, CH₂, 1H) ; 2.14 (m, CH_{2,}1H).
**IV** (KBr) (ν/cm⁻¹): 2010 (C=O); 1885 (C=O); 1690 (C=O ligando)

### 6.4. Synthesis of [Re(CO)₃(κ³-3,5-Me₂pz(CH₂)₂N(CH₂)₂(glygly) NH₂)]Br (19a)

100 mg (0.130 mmol) of (NEt₄)₂[ReBr₃(CO)₃] were mixed with 53 mg (0.130 mmol) of the ligand **13**, in water, and the solution was refluxed overnight.
Yield: 100% by ¹H-NMR
¹H-NMR (D₂O): δ 6.04 (s, H(4)pz, 1H); 5.05 (s, br, NH₂, 1H); 4.36-4.31 (m, CH_{2,} 1H); 4.16-4.04 (m, CH_{2,} 1H); 3.88 (s, NHCH₂CO, 2H); 3.84 (s, NHCH₂CO, 2H); 3.65 (s, br, NH₂, 1H); 3.53 (m, CH_{2,} 1H); 3.30 (m, CH₂, 2H); 2.86 (m, CH_{2,} 1H); 2.74 (m, CH₂, 2H); 2.57 (m, CH₂, 1H); 2.40 (m, CH₂, 1H); 2.31 (m, CH₂, 1H) ; 2.73 (s, CH₃, 3H); 2.16 (s, CH₃, 3H); 2.10 (m, CH₂, 1H) ; 1.95 (m, CH₂. 1H).

### 6.5. Synthesis of [^{99m}Tc(CO)₃(κ³-3,5-Me₂pz(CH₂)₂N(CH₂)₂ (glygly)NH₂)]⁺ (19b)

100 µl of a solution of **13** (10⁻³ M) was added to 1 ml of a solution of [^{99m}Tc(OH)₃(CO)₃]⁺ (1-2 mCi) in phosphate buffer. The solution was incubated for 1h at 100°C and then analysed by HPLC. The radiochemical purity was >90%.

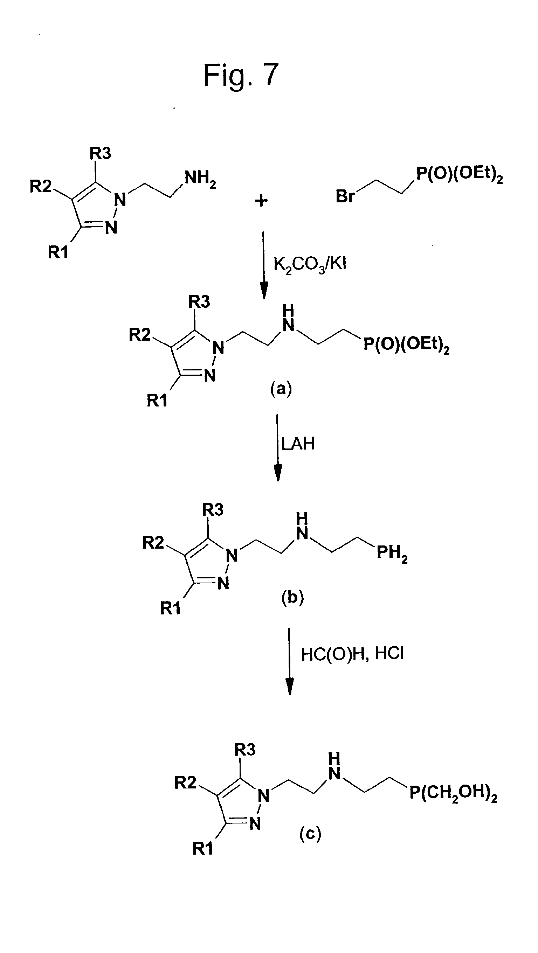

### 7. Synthesis of pyrazolyl-aminophosphines (figure 7)

The preparation of the pyrazolyl-aminophosphines of the invention involves alkylation of 1-(2-aminoethyl)pyrazoles with (2-bromoethyl)phosphonic acid diethyl esther, yielding a pyrazole-amino-phosphonate derivative (compound **a**). Reduction of compound a with lithium aluminium hydride (LAH) affords a primary phoshine (compound **b**) which is then converted to the final chelator (compound **c**) by treatment with formaldehyde in acidic medium (Katti et al., J. Am. Chem. Soc. 122, 1554 (2000).

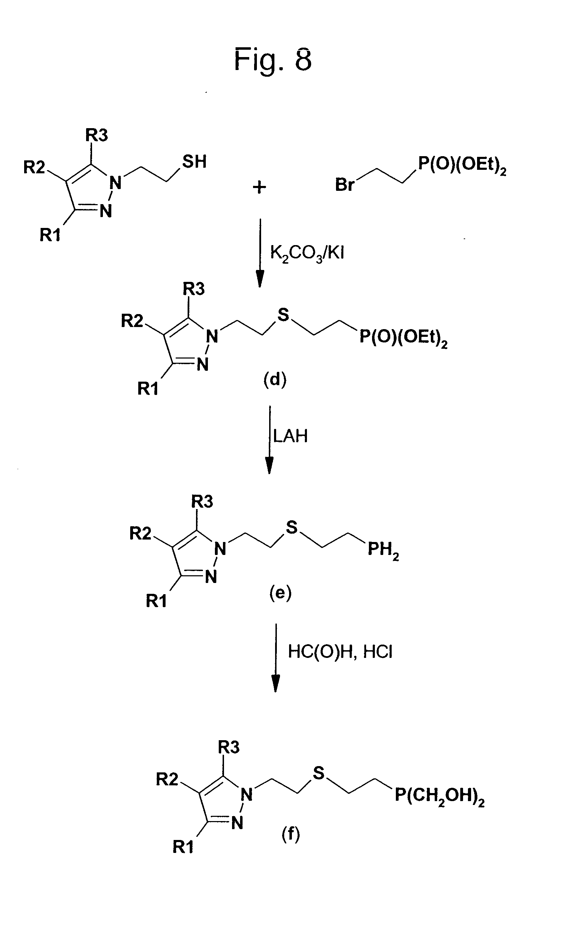

### 8. Synthesis of pyrazolyl-thioetherphosphines (figure 8)

The preparation of pyrazolyl-thioetherphosphines of the invention involves reaction of 1-(2-mercaptoethyl)pyrazoles with (2-bromoethyl)phosphonic acid diethyl esther yielding a pyrazole-thioether-phosphonate derivative (compound **d)** (Katti et al., Angew. Chem. Int. Ed. 38, 2020 (1999). Reduction of compound **d** with lithium aluminium hydride, followed by treatment of the resulting primary phoshine (compound **e**) with formaldehyde in acidic medium affords the final chelator (compound **f**).

### REFERENCES

[1]
   a) Alberto et al WO 98/48848,
   b) Alberto et al., WO 00/50086,
   c) Alberto et al., WO 01/00637
   d) Alberto et al, US 6, 344,178 B1
[2]
   a) Hilger et al, US 6, 488,909 B1.
[3]
   a) Alberto et al, Polyhedron 17 (1998) 1303
   b) Alberto et al., J. Med. Chem. 41 (1998) 4429
   c) Alberto et al, J. Am. Chem. Soc. 121 (1999) 6076
   d) Alberto et al, J. Nucl. Med. 40 (1999) 1913
   e) Schibli et al, Nucl. Med. and Biol. 26 (1999) 711
   f) Alberto et al, Bioconjugate Chem. 11 (2000) 414
   g) Alberto et al, Bioconjugate Chem. 11 (2000) 345
   h) Alberto et al., Chem. Eur. Journal 7 (2001) 1868
   i) Alberto et al, Angew. Chem. Int. Ed. 40 (2001) 3062
   j) Alberto et al, Bioconjugate Chem. 13 (2002) 750.
[4]
   a) Santos et al, J. Am. Chem. Soc. 122 (2000) 11240
   b) Santos et al., Inorg. Chem. 40 (2001) 5147
   c) Santos et al, J. Chem. Soc. Dalton Trans.(2002) 4714.
[5]
   a) Valliant et al, Inorg Chem. Commun. 41 (2002) 628
   b) Valliant et al, Inorg Chem. 41 (2002) 6417
[6]
   a) Sorrell et al, Inorg. Chem. 22 (1983) 1883
   b) Driessen et al. J. Chem. Soc. Dalton Trans. (1992) 481
   c) Parkin et al., Inorg. Chem. 35 (1996) 2415
   d) Ballesteros et al., Bioorganic and Medicinal Chem. 7 (1999) 517.
   e) Bouwman et al., Inorg. Chim. Acta (2000) 183
[7] Holzer et al, J. Heterocyclic Chem. 130 (1993) 865

## Claims

1. Chelating agent of the general formula: 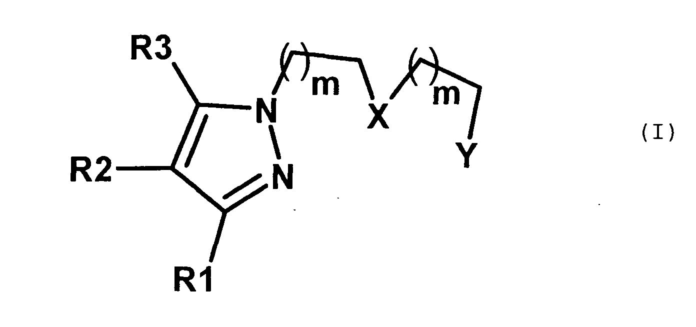 wherein
m is 0 or 1;
X is NR₄ or S;
Y is SR₅, NHR₅ or P(R₅)₂;
R₁ and R₃ are the same or different and are selected from H, alkyl or aryl;
R₂ is H, COOH, NHR₆ or (CH₂)ₙCOOR₆;
R₄ is H, alkyl, aryl, (CH₂)ₙCOOR₆ or (CH₂)ₙOR₆;
R₅ is H, alkyl, aryl, (CH₂)ₙCOOR₆ or (CH₂)ₙOR₆
R₆ is H, alkyl or aryl;
n is 1, 2, 3, 4, 5, 6, 7, 8, 9 or 10; and
when R₁=R₃=CH₃, R₂, R₄ and R₅ are not all three H.

2. Chelating agent as claimed in claim 1, wherein the alkyl is a C₁ alkyl, C₂ alkyl, C₃ alkyl, C₄ alkyl, C₅ alkyl or C₆ alkyl.

3. Chelating agent as claimed in claim 2, wherein the alkyl is methyl, ethyl, *n*-propyl, isopropyl, *n*-butyl, isobutyl, *s*-butyl, t-butyl, *n*-pentyl, isopentyl, neopentyl, *n*-hexyl, isohexyl (2-methylpentyl), neohexyl (2,2-dimethylbutyl), 3-methylpentyl, 2,3-dimethylbutyl.

4. Chelating agent as claimed in any one of the claims 1-3, wherein the aryl is monocyclic, preferably phenyl or benzyl, or polycyclic, C₁₀-C₁₈, and optionally substituted with alkyl, carboxy, oxo, amino, alkoxy or aldehyde groups.

5. Chelating agent as claimed in claim 4, wherein aryl is phenyl or benzyl.

6. Chelating agent as claimed in any one of the claims 1-5, wherein n is 2, 3, 4, 5 or 6 and preferably 2, 3 or 4.

7. Chelating agent as claimed in any one of the claims 1-6, which agent is a pyrazolyl-polyamine of the general formula: 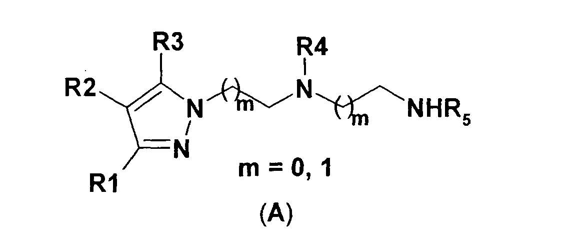 wherein R₁, R₂, R₃, R₄ and R₅ are as defined in claim 1.

8. Chelating agent as claimed in any one of the claims 1-6, which agent is a pyrazolyl-aminothioether of the general formula: 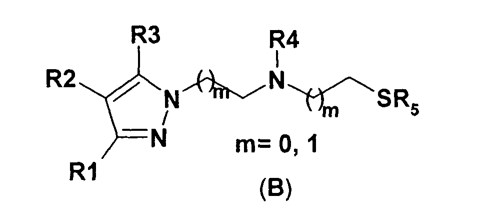 wherein R₁, R₂, R₃, R₄ and R₅ are as defined in claim 1.

9. Chelating agent as claimed in any one of the claims 1-6, which agent is a pyrazolyl-polythioether of the general formula: 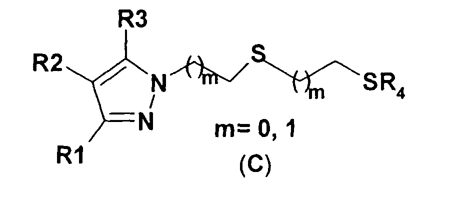 wherein R₁, R₂, R₃, R₄ and R₅ are as defined in claim 1.

10. Chelating agent as claimed in any one of the claims 1-6, which agent is a pyrazolyl-aminophosphine of the general formula: 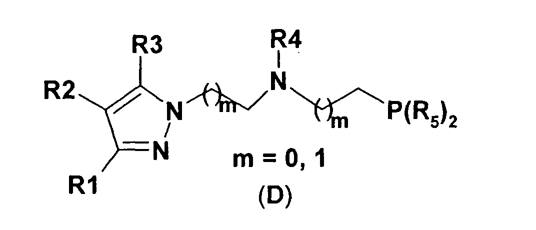 wherein R₁, R₂, R₃, R₄ and R₅ are as defined in claim 1.

11. Chelating agent as claimed in any one of the claims 1-6, which agent is a pyrazolyl-thioetherphosphine of the general formula: 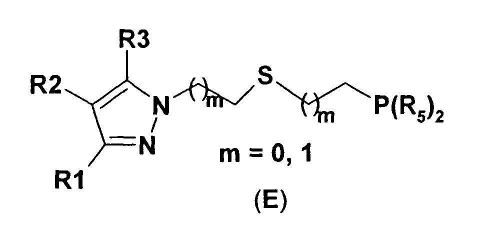 wherein R₁, R₂, R₃, R₄ and R₅ are as defined in claim 1.

12. Chelating agent as claimed in any one of the claims 1-11, wherein X and Y are N, R₆ is H, C₁ alkyl, C₂ alkyl, C₃ alkyl, C₄ alkyl, C₅ alkyl or C₆ alkyl, phenyl, benzyl or a biomolecule and R₁, R₂, R₃, R₄ and R₅ are as listed in Table 1.

13. Chelating agent as claimed in any one of the claims 1-11, wherein X and Y are S, R₆ is H, C₁ alkyl, C₂ alkyl, C₃ alkyl, C₄ alkyl, C₅ alkyl or C₆ alkyl, phenyl, benzyl or a biomolecule and R₁, R₂, R₃, R₄ and R₅ are as listed in Table 1.

14. Chelating agent as claimed in any one of the claims 1-11, wherein X is N and Y is S, R₆ is H, C₁ alkyl, C₂ alkyl, C₃ alkyl, C₄ alkyl, C₅ alkyl or C₆ alkyl, phenyl, benzyl or a biomolecule and R₁, R₂, R₃, R₄ and R₅ are as listed in Table 1.

15. Chelating agent as claimed in any one of the claims 1-11, wherein X is S and Y is N, R₆ is H, C₁ alkyl, C₂ alkyl, C₃ alkyl, C₄ alkyl, C₅ alkyl or C₆ alkyl, phenyl, benzyl or a biomolecule and R₁, R₂, R₃, R₄ and R₅ are as listed in Table 1.

16. Chelating agent as claimed in any one of the claims 1-11, wherein X is S and Y is P(R₅)₂,, R₆ is H, C₁ alkyl, C₂ alkyl, C₃ alkyl, C₄ alkyl, C₅ alkyl or C₆ alkyl, phenyl, benzyl or a biomolecule and R₁, R₂, R₃, R₄ and R₅ are as listed in Table 1.

17. Chelating agent as claimed in any one of the claims 1-11, wherein X is N and Y is P(R₅)₂, R₆ is H, C₁ alkyl, C₂ alkyl, C₃ alkyl, C₄ alkyl, C₅ alkyl or C₆ alkyl, phenyl, benzyl or a biomolecule and R₁, R₂, R₃, R₄ and R₅ are as listed in Table 1.

18. Chelating agent as claimed in any one of the claims 1-17, wherein R₆ is a biomolecule.

19. Chelating agent as claimed in claim 18, wherein the biomolecule is selected from amino acids, peptides, proteins, oligonucleotides, polynucleotides, sugars.

20. Chelating agent as claimed in claim 19, wherein the biomolecule is selected from the group consisting of antibodies, ligands of tumor receptors.

21. Chelating agent as claimed in claim 19 or 20, wherein the biomolecule is selected from the group consisting of CCK, thioglucose, glucosamine, somatostatin, neurotensin, bombesin, CCK, annexin, interleukins, growth factors, steroid hormones and molecules binding to GPIIb/IIIa receptors.

22. Chelating agent as claimed in any one of the claims 19-21, wherein the biomolecule is selected from the group consisting of glucose, thioglucose, neurotransmitters.

23. Chelating agent as claimed in any one of the claims 19-21, wherein the biomolecule is an inhibitor of the tyrosine kinase activity, such as benzothiopyranones, anilinophthalimides, quinazolines, pyridopyrimidines and pyrrolopyrimidines.

24. Chelating agent as claimed in any one of the claims 1-23, which agent is a compound of the following formula:

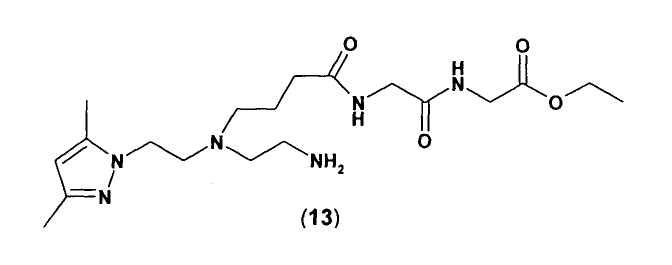

25. Chelating agent as claimed in any one of the claims 1-23, which agent is a compound of the following formula:

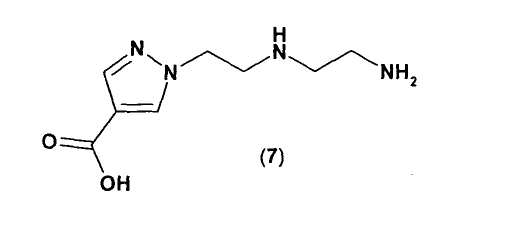

26. Chelating agent as claimed in any one of the claims 1-23, which agent is a compound of the following formula:

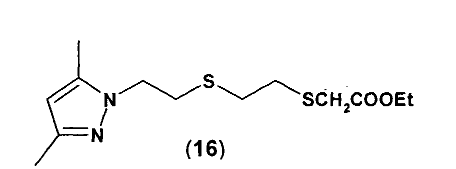

27. Chelating agent as claimed in any one of the claims 1-17, wherein the agent is complexed with a carbonyl moiety of the formula [M(CO)₃]⁺, wherein M is rhenium (Re), technetium (Tc) or manganese (Mn).

28. Chelating agent as claimed in any one of the claims 18-26, wherein the agent is complexed with a carbonyl moiety of the formula [M(CO)₃]⁺_{,} wherein M is rhenium (Re) or technetium (Tc).

29. Method for the preparation of radiolabeled biomolecules comprising:
a) contacting a chelating agent as claimed in any one of the claims 1-26 with a carbonyl moiety of the formula [M(CO)₃]⁺, wherein M is rhenium (Re) or technetium (Tc), under conditions for forming a chelator-carbonyl complex; and
b) contacting the complex with a biomolecule for obtaining a radiolabeled biomolecule.

30. Kit for performing the method as claimed in claim 29, comprising a first vial with the chelating agent of the invention, optionally a first reaction vial for contacting the chelating agent with the carbonyl moiety, a second vial with the biomolecule and optionally a second reaction vial for reacting the biomolecule with the chelator-carbonyl complex obtained in the first step of the reaction.

31. Method for the preparation of radiolabeled biomolecules comprising:
a) contacting a chelating agent as claimed in any one of the claims 1-28 with a biomolecule for obtaining a chelator-biomolecule; and
b) contacting the chelator-biomolecule with a carbonyl moiety of the formula [M(CO)₃]⁺, wherein M is rhenium (Re) or technetium (Tc), under conditions for forming a radiolabeled biomolecule.

32. Kit for performing the method as claimed in claim 31, comprising a first vial with the chelating agent of the invention, optionally a first reaction vial for reacting the chelating agent with the biomolecule, a second vial with the carbonyl moiety and optionally a second reaction vial for reacting the chelator-biomolecule obtained in the first step of the reaction with the carbonyl.

33. Chelating agent as claimed in any one of the claims 1-26 for use in the preparation of a diagnostic or therapeutic agent for diagnosing or treating tumors.

34. Chelating agent as claimed in claim 27 or 28 for use as a diagnostic or therapeutic agent for diagnosing or treating tumors.

35. Use of a chelating agent as claimed in any one of the claims 1-28 for the preparation of a diagnostic or therapeutic agent for diagnosing or treating tumors.
